(19) Europäisches Patentamt
European Patent Office
Office européen des brevets

(11) Publication number: 0 464 416 B1

## EUROPEAN PATENT SPECIFICATION

(45) Date of publication of patent specification: 07.09.94

(51) Int. Cl.⁵: **A01N 63/04**, C12N 1/14, //(A01N63/04,47:36,47:16,47:12, 43:82,43:78,43:70,43:56,43:10, 37:48,37:22,33:22),(C12N1/14, C12R1:645)

(21) Application number: 91109617.0

(22) Date of filing: 12.06.91

(83) Declaration under Rule 28(4) EPC (expert solution)

(54) New strains of Drechslera spp., and weed control agents and weed control compositions containing the same.

(30) Priority: 13.06.90 JP 152711/90

(43) Date of publication of application:
08.01.92 Bulletin 92/02

(45) Publication of the grant of the patent:
07.09.94 Bulletin 94/36

(84) Designated Contracting States:
CH DE ES FR GB IT LI NL

(56) References cited:
EP-A- 0 207 653
WO-A-90/06056
AU-D- 4 542 889

AGRICULTURAL AND BIOLOGICAL CHEMIS-
TRY, vol. 46, no. 11, 1982, pages 2681-2683,
Agricultural Chemical Society of Japan, To-
kyo, JP; D.J. ROBESON et al.: "Monocerin, a
phytotoxin from Exserohilum turcicum (=
Drechslera turcica)"

(73) Proprietor: MITSUI TOATSU CHEMICALS, Inc.
2-5 Kasumigaseki 3-chome
Chiyoda-Ku Tokyo 100 (JP)

(72) Inventor: Gohbara, Masatoshi
2142, Tougou
Mobara-shi, Chiba-ken (JP)
Inventor: Yamaguchi, Kenichi
2225, Tougou
Mobara-shi, Chiba-ken (JP)
Inventor: Shinmi, Tatsuo
2142, Tougou
Mobara-shi, Chiba-ken (JP)
Inventor: Fukui, Keiko
2785-1, Mutsuno
Mobara-shi, Chiba-ken (JP)
Inventor: Tohyama, Emi
2785-1, Mutsuno
Mobara-shi, Chiba-ken (JP)

WEED SCIENCE, vol. 33, no. 1, 1985, pages 95-99, Weed Science Society of America, Gainesville, Florida, US; R.A. KLERK et al.: "Integration of a microbial herbicide into weed and pest control programs in rice (Oryza sativa)"

SOIL BIOLOGY & BIOCHEMISTRY, vol. 20, no. 5, 1988, pages 607-612, Pergamon Press, Oxford, GB; S.-M. YU et al.: "Trifluralin concentration and the growth of Fusarium solani f. sp. cucurbitae in liquid medium and soil"

WEED SCIENCE, vol. 35, no. 3, 1987, pages 377-383, Weed Science Society of America, Gainesville, Florida, US; L.A. WYMORE et al.: "Interaction between Colletotrichum coccodes and thidiazuron for control of velvetleaf (Abutilon theopbrasti)"

PHYTOPARASITICA, vol. 16, no. 2, 1988, pages 145-152, Agricultural Research Organisation, The Volcani Center, Division of Scientific Publications, Bet Dagan, IL; G. STROBEL et al.: "The incredible fungal genus-Drechslera - and its phytotoxic ophiobolins"

(74) Representative: **VOSSIUS & PARTNER**
Postfach 86 07 67
D-81634 München (DE)

EP 0 464 416 B1

**Description**

The present invention relates to novel microorganisms and also to weed control agents and weed control compositions containing the same.

Since about 100 years ago, control methods of weeds, diseases and pests have changed from traditional cultivation control methods to chemical control methods. In particular, the rapid development of synthetic organic pesticides in the middle Nineteen Hundreds, typified by DDT, led to improvements such as increased field crop yields, improved quality and farming labor saving, so that the world food production was improved rapidly.

Serious problems have however arisen in last several years from the excessive use of such synthesized organic pesticides, including environmental pollution and the occurrence of insect pest disease and weeds with acquired pesticide resistance. In recent years, the development of chemical pesticides is therefore apparently oriented toward those having still higher activities. Namely, desired are those capable of showing excellent control effects at low application rates, i.e., dosages, to say nothing of having safety to mammals, and developed while paying sufficient attention to residuality, environmental pollution and the like. On the other hand, there is recently a growing interest in so-called pesticideless cultivation methods in which crops are cultivated without pesticides. People are now very concerned with the influence of chemical pesticides to the human body and the natural environment as mentioned above, the interests in biorational pesticides have increased so that a great deal of research and development work is under way with respect to biopesticides. A great hope is placed especially on the development of biopesticides making direct use of intact microorganisms and microbial origin pesticides using a physiologically active substance produced by a microorganism.

Yu et al. (Soil Biol. Biochem. 20(1988), 607-612) describe the plant pathogenic fungus Fusarium, which can be used as a weed control agent, and the influence of chemical herbicides on said fungus. Robeson and Strobel (Agric. Biol. Chem. 46(1982), 2681-2683) describe the phytotoxic compound Monocerin obtained from a Drechslera species.

Pesticideless cultivation methods and biological control methods are still in the middle of investigation. As a matter of fact, when applied singly, they cannot provide stable control effects so that no sufficient production of crops can be expected.

Whatever control method is used, it is very significant from the viewpoint of safety to lower the dosage of a synthetic organic pesticide.

In the field of herbicides, extensive research and development work has been carried out, centered in the United States, with respect to mycoherbicides which use a pathogen against weeds. Mycoherbicides which have been put on the market to date include "DeVine" (trade mark; product of Abbott Laboratories Ltd.) and "Collego" (trade mark; product of Ecogen Inc.). The former makes use of *Phytophthora palmiola* which is a pathogen against strangle vine (*Morrenia odorata*), an *Asclepiadaceae* weed, while the latter utilizes *Colletotrichum gloeosporioides* which is a pathogen against northern jointvetch (*Aeschynomene virginica*), a leguminous weed. The present inventors however are not aware of any investigation on mycoherbicides directed to barnyard grass (*Echinochloa spp.*) which is a troublesome weed in the cultivation of important crops such as wheat, rice and soybean.

Wild species of barnyard grass are known as weeds in the rice crop areas of the world. Barnyard grass has long been a problem as a typical lowland weed especially in Japan. Occurrence of barnyard grass in paddy fields is said to have led to the need for transplanting of rice seedlings. According to Yabuno ["Zasso Kenkyu Weed Research, Japan" 20 (1975)], barnyard grass which grows wild in the world includes *Echinochloa oryzicola, Echinochloa colonum, Echinochloa pyramidalis, Echinochloa stagnina, Echinochloa haplocloda* and *Echinochloa crus−galli*, and the *crus−galli* species is classified into three varieties, namely, *Echinochloa crus−galli var. formosensis, Echinochloa crus−galli var. crus−galli, Echinochloa crus−galli var. praticola*. Among these varieties, different reactions are observed to herbicides and pathogens. Barnyard grass which grow naturally as a weed is considered to include hybrids of these *Echinochloa* species. As a herbicide having practical control effects against barnyard grass, it must have herbicidal effects against all the species of *Echinochloa spp.*.

EP-A1 207 653 relates to herbicidal compositions comprising microbial herbicides and chemical herbicides or plant growth regulators. AU-B-45428/89 relates to weed control compositions containing various Drechslera species.

Besides barnyard grass, many weeds grow competitively with crops in lowlands and uplands. Control of these weeds is also essential in practice. To control these many weeds at once, it is the current common practice to use a few types of chemical herbicides in combination. No composition is however known to the best knowledge of the inventors, which contains in combination a microorganism having pathogenecity

3

against *Echinochloa spp*. and chemical herbicides. This may be attributed probably to the conventional belief that microorganisms generally do not have resistance to pesticides including herbicides. It has not been reported that a combination of a conventional chemical herbicide and a pathogen against *Echinochloa spp*. can lead to a synergistic increase in the herbicidal effects against *Echinochloa spp*.. This is therefore a novel technique.

Chemical herbicides useful in the practice of the present invention - such as CNP, chlormethoxynil, bifenox, mefenacet, pretilachlor, naproanilide, butachlor, propanil, clomeprop, oxadiazon, pyrazolate, bensulfuron methyl, pyrazosulfuron ethyl, benthiocarb, dimepiperate, molinate, esprocarb and simetryne etc. are either available on the market or known to the public.

An object of this invention is to overcome the above-described disadvantages in the production of crops and hence to provide a weed control agent and a weed control composition, said agent and composition being capable of preventing environmental pollution and the occurrence of resistant weeds and moreover of permitting practical weed control.

From the nature, the present inventors have isolated *Drechslera* fungi which show pathogenecity against barnyard grass only. The present inventors have also found that these *Drechslera* fungi include strains having herbicidal effects against all the *Echinochloa* species but no herbicidal effects against economical crops led by rice. It has also been found that use of a *Drechslera* strain having selective pathogenecity against barnyard grass in combination with a conventional chemical herbicide can bring about significant synergistic effects and application of the chemical herbicide at a dosage one tenth to one hundredth as little of its conventional dosage can still achieve sufficient control effects against barnyard grass. The present invention has been completed based on these findings.

The present invention therefore provides a new strain of *Drechslera monoceras*, which is free of pathogenecity against a crop under cultivation but has pathogenecity against *Echinochloa oryzicola*, *Echinochloa colonum*, *Echinochloa crus−galli var. formosensis*, *Echinochloa crus−galli var. crus−galli and Echinochloa crus−galli var. praticola*.

The present invention also provides a weed control agent comprising living fungal cells of the new strain of *Drechslera monoceras* and an agriculturally acceptable carrier. The term "carrier" as used herein should be interpreted to include not only a carrier but also a diluent.

The present invention also provides a weed control composition comprising a strain of *Drechslera spp*., a chemical herbicide and an agriculturally acceptable carrier, said strain of *Drechslera spp*. being free of pathogenecity against economic crops but having pathogenecity against *Echinochloa spp*.

The present invention can therefore reduce the dosage of a chemical herbicide while ensuring safer and sufficient control of weeds.

Barnyard grass, which is the target weed of the present invention, is a weed belonging to the genus *Echinochloa*. which in turn falls under the order of *Graminales*. Described specifically, it includes the following varieties: *Echinochloa oryzicola, Echinochloa crus−galli var. formosensis, Echinochloa crus−galli var. crus−galli, Echinochloa crus−galli var. praticola. Echinochloa colonum, Echinochloa pyramidalis, Echinochloa stagnina* and *Echinochloa haploclada*.

The microorganisms according to the present invention are new strains of *Drechslera monoceras*, which possess no pathogenecity against cultivated crops - such as rice, barley, wheat, rye, wild oat, corn, sorghum and foxtail millet - and pastures - such as orchard grass, Italian rye grass, perennial rye grass, sweet barnal grass, tall fescue, meadow fescue and meadow fescue - but have pathogenecity against *Echinochloa oryzicola, Echinochloa crus−galli, Echinochloa colonum* and the like.

Pathogens were isolated in a pure form from samples of barnyard grass, which were naturally infected. Their pathogenecity was tested against barnyard grass and rice. Based on the results of the test, those having strong pathogenecity against all the varieties of *Echinochloa spp*., which are barnyard grass weeds, but showed no pathogenecity against rice were selected. The microorganisms so selected were identified on the basis of the morphological characteristics of their conidia. They were all found to be classified under *Drechslera spp*., leading to the completion of the present invention.

The microorganisms of the present invention can be used as weed control agents in various ways, Living fungal cells obtained by culture of each microorganism can be used directly, i.e., as they are. It is also possible to use a filtrate obtained subsequent to culture of cells. As a further alternative, it is also possible to use both the living fungal cells and the filtrate as a mixture. In addition, each microorganism of the present invention can also be used either singly by suspending its conidia and hyphae, which have been obtained by culturing the microorganism on a nutrient medium, in an aqueous solution containing a surfactant or the like or in combination with one or more chemical pesticides such as other herbicides which do not exhibit competition with the microorganism, fungicides and insecticides. When the microorganisms according to the present invention are used as weed control agents, conidia having higher durability than hyphae are more desired. Although proliferation of cells of each microorganism according to the present invention is feasible whether a liquid medium or a solid medium is used, conidia can be formed by inoculating cells to a liquid medium such as a potato-dextrose medium, allowing the cells to proliferate, disrupting cells so obtained, and then drying the cells so disrupted. In the case of a solid medium, formation of conidia can be promoted by inoculating cells to a potato-dextrose-agar medium or the like, removing grown aerial hyphae and then drying cells so obtained.

The microorganisms according to the present invention permit mass production of cells such as conidia and/or hyphae as described above, so that they can be used industrially as weed control agents. Further, these weed control agents - when applied, for example, at the time of transplanting of rice seedlings to a paddy field - exhibit herbicidal effects against barnyard grass as a weed and have pathogenecity against neither cultivated crops such as rice, barley, wheat, rye, wild oat, corn, sorghum and foxtail millet nor pastures such as orchard grass, Italian rye grass, perennial rye grass, sweet barnal grass, tall fescue, meadow fescue and meadow fescue. The weed control agents therefore can provide selective herbicidal effects.

The *Drechslera* strains according to the present invention are absolutely free from biological effects of certain chemical herbicides, such as inhibition to hyphal growth and inhibition to spore germination. Their combined use can bring about substantial improvements in the herbicidal effects against barnyard grass.

Further, weed control compositions which comprise in combination at least one of the *Drechslera* fungi according to the present invention, i.e., *Drechslera monoceras*, *Drechslera ravenelii* and/or *Drechslera poae* and at least one conventional chemical herbicide make it possible to control barnyard grass at such low dosages that the conventional chemical herbicide cannot control it. Examples of the conventional chemical herbicide include oxadiazon, dimethametryne, simetryne, chlormethoxynil, dimepiperate, trifluralin, naproanilide, paraquat, pyrazoxyfen, pyrazolate, butachlor, pretilachlor, benthiocarb, mefenacet, molinate, CNP, DBN, MCP, prometryne, benzofenap, propanil, NSK-850, HW-52, clomeprop, esprocarb, bifenox, quinchlorac, buromobutide, bensulfuron methyl, pyrazosulfuron ethyl, and 2,4-D.

Regarding the mechanism of action by each weed control composition according to the present invention, it appears that absorption and transfer of the chemical herbicide may be promoted through invasive scars formed in plant tissues as a result of infection from the *Drechslera* strain and infection from the *Drechslera* strain may also be facilitated in plant tissues damaged by the chemical herbicide. The reduced dosage of the chemical herbicide owing to such synergistic action of the *Drechslera* strain and the chemical herbicides can improve today's various problems such as environmental pollution and the occurrence of resistant weeds and can substantially benefit not only agricultural producers but also general consumers.

The new strains of *Drechslera spp.*, which pertain to the present invention, have been found to have pathogenecity against all the species of *Echinochloa spp.*, i.e., barnyard grass as a weed and have shown practical herbicidal effects against barnyard grass. They have also been confirmed to be extremely safe for cultivated crops led by rice.

The microorganisms according to the present invention have been selected from microorganisms in the nature, so that they are free of the potential problem of environmental pollution by synthetic organic pesticides and are therefore safe.

The weed control compositions according to the present invention, each of which contains a *Drechslera* strain and a chemical herbicide in combination, can show sufficient herbicidal effects at dosages which are too small to control weeds when they are used singly. They have therefore made it possible to lower the dosage of chemical herbicides. The synergistic action of the *Drechslera* strains and the chemical herbicides have also made it possible to reduce the amount of conidia of the *Drechslera* strain to be applied, resulting in a reduction in the production cost.

The weed control agents and weed control compositions according to the present invention therefore contribute not only to the production of crops but also to the prevention of the today's problems, namely, environmental pollution and the occurrence of weeds with acquired pesticide resistance.

To use as herbicides the weed control agents and weed control compositions according to the present invention, cells of the *Drechslera* strain may be used as they are, together with the chemical herbicide in an undiluted form. It is however generally desirable to mix cells of the *Drechslera* strain and the undiluted chemical herbicide with an inert solid or liquid carrier and then to prepare the resultant mixture into a formulation form commonly employed in the art, such as a granular formulation, a flowable formulation, a wettable powder, an emulsion or a liquid formulation.

Any carriers can be used whether they are solid or liquid, as long as they are usually employed in agricultural and horticultural pesticides and are biologically inert.

Examples of solid carriers include mineral powders such as clay, talc, bentonite, calcium carbonate, diatomaceous earth and white carbon; vegetable flours such as soybean flour and starch; and high molecular compounds such as polyvinyl alcohol and polyalkylene glycol. On the other hand, exemplary liquid carries include various organic solvents such as decane and dodecane; vegetable oils; mineral oils; and water.

The content of the *Drechslera* strain in each weed control agent according to the present invention can, in terms of spores, be $10^2$-$10^{12}$ spores, preferably $10^6$-$10^{12}$ spores per kilogram.

The content of the chemical herbicide in each weed control composition according to the present invention varies depending on the formulation form. In general, it can be 0.05-15 wt.% in a granular formulation, 1-50 wt.% in a flowable formulation, and 1-90 wt.% in a wettable powder. Its preferred content is 0.5-8 wt.% in a granular formulation, 10-30 wt.% in a flowable formulation, and 10-50 wt.% in a wettable powder. On the other hand, the content of the *Drechslera* strain can, in terms of spores, $10^2$-$10^{12}$ spores, preferably $10^6$-$10^{12}$ per kilogram of the effective ingredients in the composition.

As adjuvants, surfactants, binders, stabilizers and the like, which are commonly used in agricultural and horticultural pesticides, can be used either singly or in combination as needed. As stabilizers, an antioxidant and/or a pH regulator may be used by way of example, A light stabilizer may also be used in some instances.

The total content of such adjuvants may range from 0 wt.% to 80 wt.%. The content of the carrier is therefore the value which is obtained by subtracting the contents of the effective ingredients and adjuvants from 100 wt.%.

When the weed control agents and weed control compositions according to the present invention are applied to a field, their dosage can, in terms of the amount of conidia of the *Drechslera* strain, be $10^2$-$10^{15}$ spores per 10 a, preferably $10^7$-$10^{12}$ spores per 10 a.

The weed control compositions according to the present invention contain one or more species of *Drechslera spp.* in combination with one or more of chemical herbicides. These weed control compositions can be used in the form of mixtures with for instance pesticides such as fungicides having no antifungal activities against *Drechslera spp.*, insecticides, plant growth regulators, fertilizers, soil improvers and their combinations.

Examples of usable chemical herbicides include: 2,4-dichloro-o-methylphenoxyacetic acid (2,4-D), 4-chlorophenoxyacetic acid (MCPA), 2,4,5-trichlorophenoxyacetic acid (2,4,5-T), 2-(2,4-dichlorophenoxy)-propionic acid (dichlorprop), 2-(2-methyl-4-chlorophenoxy)propionic acid (mecoprop), 2-(2,4,5-trichlorophenoxy)propionic acid (fenoprop), 4-(2,4-dichlorophenoxy)butyric acid (2,4-DB), 4-(2-methyl-4-chlorophenoxy)butyric acid (MCPB), 2-(2-naphthoxy)propionanilide (naproanilide), 2-(1-naphthoxy)N,N-diethylpropionamide (napropamid), methyl (±)-2-[4-(2,4-dichlorophenoxy)phenoxy]propionate (diclofop-methyl), butyl 2-[4-(5-trifluoromethyl-2-pyridyloxy)phenoxy]propionate (fluazifop), methyl 2-[4-(3-chloro-5-trifluoromethyl-2-pyridyloxy)phenoxy]propionate (haloxyfop), 2-propynyl 2-[4-(3,5-dichloro-2-pyridyloxy)-phenoxy]propionate (chlorazifop-propynyl), ethyl 2-[4-(6-chloro-2-quinoxalinyloxy)phenoxy]propionate (quizalofop-ethyl), ethyl 2-[4-(6-chloro-2-benzoxazolyl)phenoxy]propionate (fenoxaprop-ethyl), ethyl 2-[4-(6-chloro-2-benzothiazolyloxy)phenoxy]propionate (fenthiaprop-ethyl), 2,3,6-trichlorobenzoic acid (2,3,6-TBA), 3,6-dichloro-2-methoxybenzoic acid (dicamba), 2,5-dichloro-3-aminobenzoic acid (amiben), 3,5,6-trichloro-2-methoxybenzoic acid (tricamba), 4-chloro-2,2-dimethylvaleranilide (monalide), 3,4-dichloropropionanilide (propanil), 3,4-dichloro-2-methylacrylanilide (dicryl), 3,4-dichlorocyclopropane-carboxyanilide (cypromid), 3,4-dichloro-2-methylpentananilide (karsil), 3-chloro-2,4-dimethylpentananilide (solan), N-(1,1-dimethylpropynyl)-3,5-dichlorobenzamide (propyzamide), N,N-dimethyl-2,2-diphenylacetamide (diphenamide), N-naphthylphthalamic acid (naptalam), N-(1,1-dimethylbenzyl)-2-bromo-3,3-dimethylbutanamide (buromobutide), 2-benzothiazol-2-yl-oxy-N-methylacetanilide (mefenacet), N-[3-(1-ethyl-1-methylpropyl)5-isoxazolyl]-2,6-dimethoxybenzamide (isoxaben), 1,1-dimethyl-3-phenylurea (fenuron), 3-(4-chlorophenyl)-1,1-dimethylurea (monuron), 3-(4-chlorophenyl)-2,1,1-trimethylisourea (trimeturon), 3-(4-chlorophenyl)-1-methoxy-1-methylurea (monolinuron), 3-(4-chlorophenyl)-1-methyl-1-(1-methylpropyn-2-yl)urea (buturon), 3-(4-bromophenyl)-1-methoxy-1-methylurea (metobromuron), 1-(2-methylcyclohexyl)-3-phenylurea (siduron), 1,1-dimethyl-3-(3-trifluoromethylphenyl)urea (fluometuron), 3-(3,4-dichlorophenyl)-1,1-dimethylurea (diuron), 3-(3,4-dichlorophenyl)-1-methoxy-1-methylurea (linuron), 3-(3,4-dichlorophenyl)-1-n-butyl-1-methylurea (neburon), 3-(3-chloro-4-methoxyphenyl)-1,1-dimethylurea (metoxuron), 3-(4-bromo-3-chlorophenyl)-1-methoxy-1-methylurea (chlorbromuron), 3-(4-difluorochloromethylthio-3-chlorophenyl)-1,1-dimethylurea (fluothiuron), 3-(3-chloro-4-methylphenyl)-1,1-dimethylurea (chlortoluron), 3-[4-(4-chlorophenoxy)phenyl]-1,1-dimethylurea (chloroxuron), 3-[4-(4-methoxyphenoxy)phenyl]-1,1-dimethylurea (difenoxuron), 3-[3-(N-tertiary-butylcarbamoyloxy)phenyl]-1,1-dimethylurea (karbutilate), 3-benzoyl-3-(3,4-dichlorophenyl)-1,1-dimethylurea (phenobenzuron), 1-(α.α-dimethylbenzyl)-3-(4-methylphenyl)urea (dymron), 3-(4-isopropylphenyl)-1,1-

6

dimethylurea (isoproturon), 3-(2-benzothiazolyl)-1,3-dimethylurea (methabenzthiazuron), 3-(2-benzothiazolyl)-1-methylurea (benzthiazuron), 3-(hexahydro-4,7-methanoindan-5-yl)-1,1-dimethylurea (noruron), 3-cyclooctyl-1,1-dimethylurea (cycluron), 1,3-dimethyl-3-(5-trifluoromethyl-1,3,4-thiadiazol-2-yl)urea (thiazfluron), 1-(5-ethylsulfonyl-1,3,4-thiadiazol-2-yl)-1,3-dimethylurea (sulfodiazol), 3-[5-(1,1-dimethylethyl)-1,3,4-thiadiazol-2-yl]-1,3-dimethylurea (tebuthiuron), 3-(5-tertiary-butylisoxazol-3-yl)-1,1-dimethylurea (isouron), 4-[2-chloro-4-(3,3-dimethylureido)phenyl]-2-tertiary-butyl-1,3,4-oxadiazolin-5-one (dimefuron), 3-(5-tertiary butyl-1,3,4-thiadiazol-2-yl)-4-hydroxy-1-methyl-2-imidazolidinone(buthidazole), 2-chloro-4,6-bis-(ethylamino)-1,3,5-triazine (simazine), 2-chloro-4-ethylamino-6-isopropylamino-1,3,5-triazine (atrazine), 2-chloro-4,6-bis(isopropylamino)-1,3,5-triazine (propazine), 2-chloro-4-diethylamino-6-ethylamino-1,3,5-triazine (trietazine), 2-chloro-4-ethylamino-6-tertiary-butylamino-1,3,5-triazine (terbuthylazine), 2-(4-chloro-6-ethylamino-1,3,5-triazin-2-yl-amino)2-methylpropionitrile (cyanazine), 2-chloro-4-cyclopropylamino-6-isopropylamino-1,3,5-triazine (prefox), 2-[4-chloro-4-(cyclopropylamino)-1,3,5-triazin-2-yl-amino]-2-methylpropionitrile (procyazin), 6-methoxy-2-secondary-butylamino-4-ethylamino-1,3,5-triazine (secbumeton), 6-methoxy-2,4-bis-(isopropylamino)-1,3,5-triazine (prometone), 6-methylthio-2,4-bis(ethylamino)-1,3,5-triazine (simetryne), 6-methylthio-2,4-bis(isopropylamino)-1,3,5-triazine (prometryne), 6-methylthio-2-methylamino-4-isopropylamino-1,3,5-triazine (ametryne), 6-methylthio-2-ethylamino-4-tertiary-butylamino-1,3,5-triazine (terbutryn), 6-methylthio-2-isopropylamino-4-(3-methoxypropylamino)-1,3,5-triazine (methoprotryne), 6-methylthio-2-(1,2-dimethylpropylamino)-4-ethylamino-1,3,5-triazine (dimethametryne), 6-methylthio-2-isopropylamino-4-methylamino-1,3,5-triazine (desmetryne), 4-amino-6-tertiary-butyl-3-methylthio-1,2,4-triazin-5(4H)-one (metribuzin), 2-ethylthio-4,6-bis(isopropylamino)-1,3,5-triazine (dipropetryn), 2-tertiary-butylamino-4-ethylamino-6-methoxyamino-1,3,5-triazine (terbumeton), 2-azide-4-isopropylamino-6-methylthio-1,3,5-triazine (aziprotryne), 4-amino-3-methyl-6-phenyl-1,2,4-triazin-5(4H)-one (metamitron), 6-tertiary-butyl-4-isobutylideneamino-1,2,4-triazin-5(4H)-one (isomethiozin), 3-cyclohexyl-6-dimethylamino-1-methyl-1,3,5-triazin-2,4-(1H,3H)-dione (hexazinone), ethyl-N-(4-chloro-6-ethylamino-1,3,5-triazin-2-yl)-aminoacetate (eglinazine), ethyl-N-(4-chloro-6-isopropylamino-1,3,5-triazin-2-yl)-aminoacetate (proglinazine), 2-chloro-N-isopropylacetanilide (propachlor), N-methoxymethyl-2',6'-diethyl-2-chloroacetanilide (alachlor), 2-chloro-2',6'-diethyl-N-(buthoxymethyl)acetanilide (butachlor), 2-chloro-2'-ethyl-6'-methyl-N-(2-methoxy-1-methylethyl)-acetanilide (metolachlor), N,N-diaryl-2-chloroacetamide (allidochlor), 2-chloro-2',6'-dimethyl-N-(2-methoxyethyl)acetanilide (dimethachlor), 2,6-dinitro-N,N-dipropyl-4-trifluoromethylaniline (trifluralin), N-butyl-N-ethyl-2,6-dinitro-4-trifluoromethylaniline (benfluralin), 2,6-dinitro-N-propyl-N-cyclopropyl-4-trifluoromethylaniline (profluralin), N,N-diethyl-2,4-dinitro-6-trifluoromethyl-m-phenylenediamine (dinitramin), 4-isopropyl-2,6-dinitro-N,N-dipropylaniline (isopropaline), 2,6-dinitro-N-secondary-butyl-4-tertiary-butylaniline(butralin), 4-methylsulfonyl-2,6-dinitro-N,N-dipropylaniline (nitralin), 3,4-dimethyl-2,6-dinitro-N-1-ethylpropylaniline (pendimethalin), 3,5-dinitro-4-dipropyl-aminobenzensulfonamide (oryzalin), N-ethyl-N-(2-methyl-allyl-2,6-dinitro-4-(trifluoromethyl)aniline (ethalfluralin), N-N-diethyl-2,4-dinitro-6-trifluoromethyl-m-phenylenediamine (diethamine), 2-chloro-N-(4-methoxy-6-methyl-1,3,5-triazin-2-yl-aminocarbonyl)benzenesulfonamide (chlorsulfuron), methyl 2-[3-(4-methoxy-6-methyl-1,3,5-triazin-2-yl)ureidosulfonyl]benzoate (metsulfuron-methyl), methyl 2-[3-(4,6-dimethoxypyrimidin-2-yl) ureidosulfonylmethyl]benzoate (bensulfuron), ethyl 2-[3-(4-chloro-6-methoxypyrimidin-2-yl)ureidosulfonyl]benzoate (chlorinuronethyl), methyl 3-[3-(4-methoxy-6-methyl-1,3,5-triazin-2-yl)ureidosulfonyl]-thiophenecarboxylic acid (thiameturon), Ethyl 5-[3-(4,6-dimethoxypyrimidin-2-yl)-ureidosulfonyl]-1-methylpyrazole-4-carboxylate (pyrazosulfuron ethyl), 3-(4,6-dimethoxy-1,3,5-triazin-2-yl)-1-[2-(2-methoxyethoxy)-phenylsulfonyl]urea (esnosufuron), 3,7-dichloro-8-quinolinecarboxylic acid (quinchlorac), 3,6-dichloro-2-pyridinecarboxylic acid (clopyralid), α-(2-chlorophenyl)-α-(4-chlorophenyl)-5-pyrimidinemethanol (fenarimol), S,S-dimethyl-2-(difluoromethyl)-4-(2-methylpropyl)-6-trifluoromethyl-3,5-pyridinedicarbo-thioate (dithiopyr), 4-chloro-5-(methylamino)-2-(3-trifluoromethylphenyl)-3(2H)-pyridazinone (norflurazon), O,O-bis(1-methylethyl)-S-[2-(phenylsulfonyl)aminoethyl]phosphorodithioate (bensulide), ( + )-2-[4,5-dihydro-4-methyl-4-(1-methylethyl)-5-oxo-1H-imidazole-2-yl]-5-ethyl-3-pyridinecarboxylic acid (imazaethapyr), 3-[5-(1,1-dimethylethyl)-3-isoxazolyl)-4-hydroxy-1-methyl-2-imidazolidione (busoxinone), 2-[1-(ethoxyimino)butyl]-3-hydroxy-5-(2H-tetrahydrothiopyran-3-yl)-2-cyclohexene-1-one (cycloxydim), S-(4-chlorobenzyl)N-N-diethylthiocarbamate (benthiocarb), S-etnyl N,N-hexamethylenethiocarbamate (molinate), isopropyl N-phenylcarbamate (propham), isopropyl N-(3-chlorophenyl)carbamate (chloro propham), methyl N-(3,4-dichlorophenyl)carbamate (swep), 3-(ethoxycarbonylamino)phenyl N-phenylcarbamate (desmedipham), 3-(methoxycarbonylamino)phenyl N-(3-methylphenyl)carbamate (phenmedipham), S-2,3-dichloro-2-propenyl N,N-diisopropylthicarbamate (diallate), S-ethyl N,N-di-n-propylthiocarbamate (EPTC), S-ethyl N-cyclohexyl-N-ethyltiocarbamate (cycloate), methyl N-(4-aminobenzenesulfony)carbamate (asulam), S-α,α-dimethylbenzyl)piperidine-1-carbothioate (dimepiperate), S-benzyl N-ethyl-N-(1,2-dimethylpropyl)-thiocarbamate (esprocarb), O-(3-tert-buthylphenyl) N-(6-methoxy-2-pyridyl)-N-methyl thiocarbamate (pributycarb), 2,4-dichlorophenyl-3-methoxy-4-nitrophenylether (chlomethoxynil), 2,4,6-trichlorophenyl-4-

7

nitrophenylether (CNP), methyl-5-(2,4-dichlorophenoxy)-2-nitrobenzoate (bifenox), sodium 5-(2-chloro-4-trifluoromethylphenoxy)-2-nitrobenzoate (acifluorfensodium), 1-ethoxycarbonylethyl-5-(2-chloro-4-trifluoromethylphenoxy)-2-nitrobenzoate (lactofen), 5-(2-chloro-4-trifluoromethylphenoxy)-N-methanesulufonyl-2-nitrobenzamido (fomesafen), 2-chloro-2',6'-diethyl-N-(n-propoxyethyl)-acetanilide (pretilachlor), 2-(2,4-dichloro-3-methylphenoxy)-propionanilide (clomeprop), 5-tert-butyl-3-(2,4-dichloro-5-isopropoxyphenyl)-1,3,4-oxadiazol-2(3H)-one (oxadiazon), 2-(3',4'-dichlorophenyl)-4-methyl-1,2,4-oxadiazolidine-3,5-dione (methazole), 3-isopropyl-1H-2,1,3-benzothiadiazin-4-(3H)one-2,2-dioxide (bentazone), 4-(2,4-dichlorobenzoyl)-1,3-dimethyl-1H-pyrazol-5-yl-p-toluenesulfonate (pyrazolate), 4-(2,4-dichlorobenzyl)-1,3-dimethyl-5-phenacyloxy-1H-pyrazole (pyrazoxyfen), 4-(2,4-dichloro-3-methylbenzyl)-1-,3-dimethyl-5-(4-methylphenacyloxy)-1H-pyrazole (benzofenap), 2-(4-isopropyl-4-methyl-5-oxo-imidazolin-2-yl)-3-quinolinic acid (imazaquin), and 2-chloro-N-(3-methoxy-2-thienyl)methyl-2',6'-dimethylacetanilide (NSK-850).

In particular, when weed control compositions according to the present invention contain one or more herbicides for barnyard grass, for example, one or more chemical herbicides such as diphenyl ether herbicides, anilide herbicides or thiolcarbamate herbicides, it is possible to obtain synergistic herbicidal effects which are so large that they cannot be expected when these herbicides are applied individually. Thus, it is possible to control barnyard grass at an unexpectedly low herbicide dosage.

Further, when weed control compositions according to the present invention contain one or more herbicides for broad-leaf weeds, for example, one or more chemical herbicides such as sulfonylurea herbicides or triazine herbicides, it is possible to obtain synergistic herbicidal effects which are so large that they cannot be expected when these herbicides are applied individually. It is therefore possible to reduce the dosage of the pathogen, one of the two effective ingredients, and also to lower the dosage of the chemical herbicide. In addition, various lowland weeds can be controlled owing to the broad-spectrum herbicidal effects of the compositions.

Certain weed control compositions according to the present invention, each of which comprises a *Drechslera* strain and a chemical herbicide, will hereinafter be described specifically by the following tests and examples.

Test 1

Isolation and Selection Method of Microorganisms and Their Identification

1) Isolation method of pathogens

Naturally-infected barnyard grass was collected. Centering at individual lesions, leaf pieces of 10-20 mm long were cut off. Those leaf pieces of barnyard grass were dipped for 1-2 seconds in a 70% ethyl alcohol solution and then for 10 minutes in a sodium hypochlorite solution having an effective chlorine concentration of 2%, whereby the leaf pieces of barnyard grass were subjected to surface sterilization. The surface-sterilized lesion tissues were washed three times with distilled water and then placed on a nutrient-free agar medium. Static culture was carried out at 25°C for 72 hours. After the culture, hyphae tips of mold fungi thus grown were subjected to single hyphae isolation under a stereoscopic microscope, followed by purification on a nutrient medium to obtain more than 5,000 strains. Of these, the promising strains, MH-0015, MH-0042, MH-0060, MH-0122, MH-1889, MH-2653, MH-2679, MH-2781, MH-2883, MH-2895, MH-4415, MH-4418, MH-5011, MH-5017, MH-5018, MH-5511 and MH-9011 strains have been deposited under the Budapest treaty with Fermentation Research Institute, Agency of Industrial Science and Technology, Ministry of International Trade and Industry (FRI), 1-3 Higashi 1-chome, Tsukubashi, Ibaraki-ken 305, Japan. Their deposit numbers and the like are shown in a list to be described subsequently. With respect to the mold fungi so isolated, their pathogenecity against barnyard grass and their safety to rice were tested.

2) Tests for pathogenecity of the isolated microorganisms against barnyard grass and their safety to rice

Barnyard grass and rice (varieties: Nipponbare) were allowed to aseptically grow in test tubes to provide test samples. Namely, barnyard grass seeds and rice seeds were dipped for 1-2 seconds in a 70% ethyl alcohol solution and then for 10 minutes in a sodium hypochlorite solution having an effective chlorine concentration of 2%, whereby the seeds were subjected to surface sterilization. The seeds so surface-sterilized were then washed three times with distilled water. The sterilized seeds were then planted in test tubes which contained a pre-sterilized, nutrient-free liquid medium. They were allowed to grow to the 1.5 leaf stage in a plant growth chamber.

On the other hand, the isolated microorganisms were individually subjected to plate culture in petri dishes containing potato-dextrose agar medium, respectively. Colonies were punched out along their circumferences by a sterilized cork borer to obtain mycelial discs as seed cell sources.

The mycelial discs were separately placed on the liquid culture medium in the test tubes in which barnyard grass seedlings and rice seedlings were grown. After incubating them for 10 days in a plant growth chamber, the pathogenecity of each microorganism against barnyard grass and rice was evaluated in accordance with the following 4-stage system ranging from - to + + +. The results are summarized in Table 1.

+ + + Death

+ + Severe inhibition to the growth

+ Some inhibition to the growth

- No effect

Incidentally, MH-0003, MH-0007 and MH-0011 strains are *Drechslera, Phoma* and *Fusarium* strains, respectively. They were all tested for comparison.

Table 1

| Pathogenecity of Isolated *Drechslera spp.* | | |
|---|---|---|
| Microorganism | Barnyard grass | Rice |
| MH-0003 | - | - |
| MH-0007 | - | + + + |
| MH-0011 | + + + | + + + |
| MH-0015 | + + + | - |
| MH-0042 | + + + | - |
| MH-0060 | + + + | - |
| MH-0122 | + + + | - |
| MH-1889 | + + + | - |
| MH-2653 | + + + | - |
| MH-2679 | + + + | - |
| MH-2781 | + + + | - |
| MH-2883 | + + + | - |
| MH-2895 | + + + | - |
| MH-2990 | + + + | - |
| MH-2998 | + + + | - |
| MH-4415 | + + + | - |
| MH-4418 | + + + | - |
| MH-5011 | + + + | - |
| MH-5017 | + + + | - |
| MH-5018 | + + + | - |
| MH-5511 | + + + | - |
| MH-9011 | + + + | - |
| Untreated | - | - |

3) Identification of microorganisms

Identification was conducted with respect to the strains which exhibited marked pathogenecity against barnyard grass but were not recognized at all to have effects on rice. As a result, each of MH-0015, MH-1889, MH-2653, MH-2679, MH-4415, MH-4418, MH-5011, MH-5017, MH-5018, MH-5511 and MH-9011 strains gave a colony as large as 65-75 mm in diameter and showed irregular growth when subjected to plate culture at 28°C for 7 days on a malt-agar medium. Those colonies had a grayish black color. Conidia had scars and their sizes were 15-17.5 $\mu$m in width and 87.5-127.5 $\mu$m in length. They had a somewhat bent shape. The conidia had 5-7 septa mostly. Conidiophores had a straight shape. From the above characteristics, MH-0015, MH-1889, MH-2653, MH-2679, MH-4415, MH-4418, MH-5011, MH-5017, MH-5018, MH-5511 and MH-9011 strains were all identified as strains of *Drechslera monoceras*.

MH-0042, MH-0060 and MH-2883 strains had conidia which contained no scar and had a size of 17-22 $\mu$m in width and 40-90 $\mu$m in length. Regarding the shape of the conidia, some bent, cylindrical conidia were observed. The conidia contained 1-5 septa. From the foregoing characteristics, MH-0042, MH-0060 and MH-2883 strains were identified as strains of *Drechslera ravenelii*.

Each of MH-0122, MH-2781 and MH-2895 strains gave a colony as large as 20-25 mm in diameter when subjected to plate culture at 28°C for 7 days on a malt-agar medium. The colony had a bright gray color, but was grayish green at a central part thereof and grayish black on the back. Its conidia were free of scars and had a size of 20-25 $\mu$m in width and 55-95 $\mu$m in length. The conidia contained 5-6 septa. Conidiophores had a straight shape. From the above characteristics, MH-0122, MH-2781 and MH-2895 strains were identified as strains of *Drechslera poae.*

Further, MH-2990 and MH-2998 strains were identified as strains of *Drechslera spp.* in view of the shapes of their colonies and conidia.

On the other hand, MH-0003 strain, MH-0011 strain and MH-0007 strain, which did not show selective pathogenecity against barnyard grass only, but not on rice, were identified as a strain of *Drechslera spp.*, a strain of *Fusarium spp.* and a strain of *Phoma spp.*, respectively.

The above identification was conducted with reference to M.B. Ellis, "Demariaceus Hyphomycetes", 608 Commonwealth Mycological Institute, Kew, England (1971) and M.B. Ellis, "More Demariaceus Hyphomycetes", 507, Commonwealth Mycological Institute, Kew, England (1976).

The *Drechslera* strains according to the present invention are not described as pathogens in the Pathogens Safety Control Guideline compiled by the National Institutes of Health (Japan) and are believed to be safe to mammals.


Test 2


Control Effects of *Drechslera* Strains against Barnyardgrass


Each *Drechslera* strain isolated from the nature inoculated on an oatmeal-agar medium, followed by static culture at 25°C for 7 days. Aerial hyphae were then removed with distilled water to promote formation of conidia. The conidia so obtained were suspended in a 0.02% "Triton X-100" (trade name; product of Rohm & Haas Co.) solution to give concentrations of $10^8$ spores/m$\ell$ and $10^5$ spores/m$\ell$, thereby preparing weed control agents containing the *Drechslera* strain as an effective ingredient.

On the other hand, barnyard grass and rice (varieties: "Nipponbare") were seeded in lowland soil which was contained in 1/10000-are pots, and were reared to the 1.5 leaf stage respectively. After the pots were irrigated to keep the seedlings under submerged conditions of about 3 cm in water depth, the above weed control agents containing conidia of the *Drechslera* strain were separately applied dropwise in an amount of 5 m$\ell$ per pot. After the seedlings were reared for 10 days in a weather-controlled room which was maintained at 30°C during the daytime and at 25°C at night, effects of the *Drechslera* strain on barnyard grass and rice were evaluated in accordance with a similar standard to Test 1. The results are shown in Table 2.

+ + +     Death  
+ +      Severe inhibition to the growth  
+       Some inhibition to the growth  
-       No effect

EP 0 464 416 B1

Table 2

| Selective Herbicidal Activities of *Drechslera spp.* - Pot Test | | | | |
|---|---|---|---|---|
| Microorganisms | Barnyard grass | | Rice | |
| | $10^8$ | $10^5$ | $10^8$ | $10^5$ |
| MH-0015 | + + | + | - | - |
| MH-0042 | + + | - | - | |
| MH-0060 | + + | - | - | - |
| MH-0122 | + + | - | - | - |
| MH-1889 | + + + | + | - | - |
| MH-2653 | + + | + | - | - |
| MH-2679 | + + | - | - | - |
| MH-2781 | + + | - | - | - |
| MH-2883 | + + | - | - | - |
| MH-2895 | + + | - | - | - |
| MH-2990 | + + | - | - | - |
| MH-2998 | + + | - | - | - |
| MH-4415 | + + + | + + + | - | - |
| MH-4418 | + + + | + + + | - | - |
| MH-5011 | + + + | + + + | - | - |
| MH-5017 | + + + | + + + | - | - |
| MH-5018 | + + + | + + + | - | - |
| MH-5511 | + + + | + + + | - | - |
| MH-9011 | + + + | + + + | - | - |
| Untreated | - | - | - | - |

As a result of the test, MH-4415, MH-4418, MH-5011, MH-5017, MH-5018, MH-5511 and MH-9011 strains of *Drechslera spp.*, said strains all pertaining to the present invention, showed herbicidal effects against barnyard grass, which were as much as 100-1,000 times superior to MH-0015 to MH-2998 strains. Further, their safety to rice was also observed.

Test 3

In a similar manner to Tests 1-2, pathogenecity of each microorganism according to the present invention against various species of *Echinochloa spp.* and rice was evaluated. Tested as barnyard grass were *Echinochloa colonum*, *Echinochloa oryzicola, Echinochloa crus−galli var. formosensis*, *Echinochloa crus−galli var. crus−galli* and *Echinochloa crus−galli var. praticola*. Tested as rice were "Nipponbare", "Sasanishiki" and "Koshihikari", which are rice species cultivated. Pathogenecity of each microorganism according to the present invention against the barnyard grass and rice was evaluated 10 days after the inoculation. The results are shown in Table 3.

## Table 3

### Pathogenecity of Invention Microorganisms against Various _Echinochloa spp._

| Microorganism | Barnyard grass | | | | | Rice | | |
|---|---|---|---|---|---|---|---|---|
| | Echinochloa oryzicola | Echinochloa crus-galli var. formosensis | Echinochloa crus-galli var. crus-galli | Echinochloa crus-galli var. praticola | Echinochloa colonum | Nipponbare | Sasanishiki | Koshihikari |
| MH0015 | - | +++ | +++ | +++ | +++ | - | - | - |
| MH2653 | + | +++ | +++ | +++ | +++ | - | - | - |
| MH4418 | +++ | +++ | +++ | +++ | +++ | - | - | - |
| MH5011 | +++ | +++ | +++ | +++ | +++ | - | - | - |
| MH5017 | +++ | +++ | +++ | +++ | +++ | - | - | - |
| MH5018 | +++ | +++ | +++ | +++ | +++ | - | - | - |
| MH5511 | +++ | +++ | +++ | +++ | +++ | - | - | - |
| MH9011 | +++ | +++ | +++ | +++ | +++ | - | - | - |
| Untreated | - | - | - | - | - | - | - | - |

+++: Death,    ++:  Marked inhibition to the growth,    +: Some inhibition to the growth,    -: No effect

As a result of the test, MH-4418, MH-5011, MH-5017, MH-5018, MH-5511 and MH-9011 strains of _Drechslera monoceras_, said strains pertaining to the present invention, had high pathogenecity against all the species and varieties of _Echinochloa spp._ and showed excellent herbicidal effects against them. Further, their safety to rice was also observed.

EP 0 464 416 B1

Test 4

Effects of Chemical Herbicides on Various Biological Properties of *Drechslera spp.*

Mycelial discs of each strain of *Drechslera spp.*, said discs having been prepared by the method described in Test 1-2), were separately placed on layers of a potato-dextrose agar medium, which contained chemical herbicides such as CNP (Herbicide A in tables), mefenacet (Herbicide E in tables), pretilachlor (Herbicide I in tables), benthiocarb (Herbicide L in tables) and bensulfuron (Herbicide P in tables) at the concentration of 500 ppm, respectively. Static culture was then conducted at 25°C for 5 days. The diameter of each colony so formed was measured and was recorded as a hypha length. Further, spores in aqueous "Triton X-100" solutions of spore suspensions of each strain of *Drechslera spp.*, said solutions having been prepared by the method described in Test 2, were resuspended in portions of a potato-dextrose liquid medium, which contained the individual chemical herbicides at the concentration of 500 ppm, respectively. Shaking culture was then conducted at 25°C for 24 hours. After the culture, spore germination was microscopically observed to calculate the rate of spore germination. Effects of each chemical herbicide on the growth of hyphae and the rate of spore germination of each strain of *Drechslera spp.* are expressed in terms of percentage relative to a corresponding control group which did not contain the chemical herbicide. The results are shown in Tables 4-1 and 4-2.

As is apparent from Tables 4-1 and 4-2, neither hyphal growth inhibition nor spore germination inhibition by the chemical herbicides was practically observed with respect to the *Drechslera* strains according to the present invention.

Table 4-1

| Effects of Chemical Herbicides against Hyphal Growth of *Drechslera spp.* | | | | |
|---|---|---|---|---|
| Microorganism | Chemical herbicide | | | |
| | A | E | I | L |
| MH-0015 | 98 | 96 | 85 | 93 |
| MH-0042 | 99 | 98 | 83 | 93 |
| MH-0060 | 101 | 95 | 84 | 92 |
| MH-0122 | 103 | 98 | 87 | 98 |
| MH-1889 | 100 | 100 | 90 | 100 |
| MH-2653 | 99 | 95 | 86 | 96 |
| MH-2679 | 100 | 96 | 84 | 95 |
| MH-2781 | 103 | 98 | 88 | 99 |
| MH-2883 | 105 | 100 | 88 | 98 |
| MH-2895 | 102 | 100 | 86 | 96 |
| MH-2990 | 99 | 94 | 86 | 97 |
| MH-2998 | 98 | 96 | 82 | 93 |
| MH-4415 | 100 | 100 | 98 | 100 |
| MH-4418 | 98 | 98 | 88 | 100 |
| MH-5011 | 96 | 100 | 92 | 100 |
| MH-5017 | 100 | 98 | 90 | 98 |
| MH-5018 | 100 | 100 | 95 | 100 |
| MH-5511 | 100 | 100 | 98 | 100 |
| MH-9011 | 98 | 98 | 90 | 100 |

Table 4-2

| Effects of Chemical Herbicides against Spore Germination of *Drechslera spp.* | | | | | |
|---|---|---|---|---|---|
| Microorganism | Chemical herbicide | | | | |
| | A | E | I | L | P |
| MH-0015 | 95 | 97 | 88 | 83 | 100 |
| MH-0042 | 98 | 98 | 85 | 85 | 99 |
| MH-0060 | 99 | 94 | 84 | 84 | 101 |
| MH-0122 | 100 | 99 | 86 | 87 | 98 |
| MH-1889 | 98 | 100 | 90 | 92 | 98 |
| MH-2653 | 100 | 95 | 88 | 85 | 103 |
| MH-2679 | 99 | 97 | 85 | 86 | 100 |
| MH-2781 | 97 | 99 | 89 | 88 | 96 |
| MH-2883 | 102 | 98 | 88 | 89 | 101 |
| MH-2895 | 99 | 100 | 87 | 88 | 104 |
| MH-2990 | 100 | 96 | 88 | 86 | 98 |
| MH-2998 | 98 | 98 | 89 | 85 | 102 |
| MH-4415 | 101 | 98 | 90 | 95 | 100 |
| MH-4418 | 100 | 98 | 98 | 90 | 102 |
| MH-5011 | 98 | 92 | 90 | 88 | 98 |
| MH-5017 | 100 | 98 | 95 | 87 | 99 |
| MH-5018 | 100 | 98 | 90 | 85 | 95 |
| MH-5511 | 105 | 100 | 95 | 90 | 100 |
| MH-9011 | 102 | 100 | 98 | 95 | 102 |

Test 5

Control Effects of Compositions of *Drechslera* Strain and Chemical Herbicide CNP on Barnyard Grass

Each *Drechslera* strain isolated from the nature was inoculated to an oatmeal agar medium, followed by static culture at 25°C for 7 days. Aerial hyphae were then removed with distilled water to promote formation of conidia. The conidia so obtained were suspended in a 0.02% "Triton X-100" (trade name; product of Rohm & Haas Co.) solution to give prescribed concentrations, thereby preparing weed control agents containing the *Drechslera* strain as an effective ingredient.

Regarding CNP, its 9% granular formulation was weighed in the amounts of 30-1 mg to use it as a chemical herbicide.

On the other hand, barnyard grass and rice (varieties: "Nipponbare") were seeded in lowland soil which was contained in 1/10000-are pots, and were reared to the 1.5 leaf stage respectively. After the pots were irrigated to keep the seedlings under submerged conditions of about 3 cm in water depth, the above weed control agents containing conidia of the *Drechslera* strain were separately applied dropwise in an amount of 5 mℓ per pot. At the same time, each pot was treated with the chemical herbicide CNP. After the seedlings were reared for 10 days in a weather-controlled room which was maintained at 30°C during the day time and at 25°C at night, effects of the *Drechslera* strain and the chemical herbicide on the barnyard grass and rice were evaluated in accordance with a similar standard to Test 1. The results are shown in Table 5.

+ + +     Death
+ +       Severe inhibition to the growth
+         Some inhibition to the growth
-         No effect

Although the results of Table 5 are only one example, the *Drechslera* strain and the chemical herbicide exhibited significant synergistic effects in each combination.

14

Table 5

| Synergistic Effects of *Drechslera spp.* and Chemical Herbicides | | | | | |
|---|---|---|---|---|---|
| Microorganism /1/10000 are | C N P (mg) | | | | |
| | 30 | 10 | 3 | 1 | 0 |
| $1 \times 10^6$ spores | 100 (-) | 100 (-) | 100 (-) | 100 (-) | 100 (-) |
| $3 \times 10^5$ spores | 100 (-) | 100 (-) | 100 (-) | 100 (-) | 80 (-) |
| $1 \times 10^5$ spores | 100 (-) | 100 (-) | 100 (-) | 100 (-) | 50 (-) |
| $3 \times 10^4$ spores | 100 (-) | 100 (-) | 100 (-) | 70 (-) | 35 (-) |
| $1 \times 10^4$ spores | 100 (-) | 90 (-) | 60 (-) | 50 (-) | 15 (-) |
| 0 spore | 100 (-) | 60 (-) | 35 (-) | 20 (-) | 0 (-) |
| Barnyard grass control value: 0-100% <br> Sign in parentheses indicates injury against rice. | | | | | |

Formulation examples and herbicidal activity tests of weed control compositions according to the present invention will next be described.

Formulation Example 1 (Granular formulation)

After 2 wt.% of "Neopelex" (trade mark; product of Kao Corporation), 2 wt.% of "Sun Ekis P252" (trade name; product of Sanyo-Kokusaku Pulp Co., Ltd.) and 96 wt.% of zeolite were thoroughly mixed, a spore suspension containing $10^9$ conidia of *Drechslera* MH-5018 per gram of a granular formulation to be formulated was added to the resultant mixture to moisten the same. The thus-moistened mass was then extruded into granules by a small extruder. The granules were dried in air, crushed and then processed by a shifting machine, whereby granules of 0.3-2 mm were obtained.

Formulation Example 2 (Granular formulation)

After 2 wt.% of "Neopelex" (trade mark; product of Kao Corporation), 2 wt.% of "Sun Ekis P252" (trade name; product of Sanyo-Kokusaku Pulp Co., Ltd.) and 96 wt.% of zeolite were thoroughly mixed, a spore suspension containing $10^8$ conidia of *Drechslera* MH-5511 per gram of a granular formulation to be formulated was added to the resultant mixture to moisten the same. The thus-moistened mass was then extruded into granules by a small extruder. The granules were dried in air, crushed and then processed by a shifting machine, whereby granules of 0.3-2 mm were obtained.

Formulation Example 3 (Granular formulation)

After 2 wt.% of "Neopelex" (trade mark; product of Kao Corporation), 2 wt.% of "Sun Ekis P252" (trade name; product of Sanyo-Kokusaku Pulp Co., Ltd.) and 96 wt.% of zeolite were thoroughly mixed, a spore suspension containing $10^9$ conidia of *Drechslera* MH-9011 per gram of a granular formulation to be formulated was added to the resultant mixture to moisten the same. The thus-moistened mass was then extruded into granules by a small extruder. The granules were dried in air, crushed and then processed by a shifting machine, whereby granules of 0.3-2 mm were obtained.

Formulation Example 4 (Wettable powder)

A mixture of 2 wt.% of "Neopelex" (trade mark; product of Kao Corporation), 2 wt.% of Triton (X-100) and 5 wt.% of white carbon was impregnated with a spore suspension containing $10^9$ conidia of *Drechslera* MH-4418 per gram of a wettable powder to be formulated, followed by drying in air. Diatomaceous earth (91 wt.%) was then added, followed by thorough mixing and grinding to obtain the wettable powder.

Formulation Example 5 (Wettable powder)

A mixture of 2 wt.% of "Neopelex" (trade mark; product of Kao Corporation; sodium dodecylbenzenesulfonate), 1 wt.% of "Neugen EA 80" (trade name, product of Sanyo Chemical Industries, Ltd.;

EP 0 464 416 B1

polyoxyethylenenonylphenyl ether), 5 wt.% of white carbon and 92 wt.% of diatomaceous earth was impregnated with a spore suspension containing $10^9$ conidia of *Drechslera* MH-5511 per gram of a wettable powder to be formulated, followed by drying in air. The resultant mixture was thoroughly mixed and ground to obtain the wettable powder.

Formulation Example 6 (Flowable formulation)

"Sun Ekis P252" (10 wt.%) dissolved in 80 wt.% of water was wet-ground, followed by the addition of 0.4 wt.% of "Kelzan S" (trade name; product of Kelco Corp.) dissolved in 9.6 wt.% of a spore suspension which contained $10^{10}$ conidia of *Drechslera* MH-5017 per gram of a flowable formulation to be obtained. The resultant mixture was mixed to obtain the flowable formulation.

Formulation Example 7 (Flowable formulation)

After 10 wt.% of "Sun Ekis P252" (trade name; described above) dissolved in 70 wt.% of water was mixed with 10 wt.% of a spore suspension which contained $10$ conidia of *Drechslera* MH-5511 per gram of a flowable formulation to be obtained, the resultant mixture was wet-ground. Then, 0.2 wt.% of "Kelzan S" (trade name; product of Kelco Corp.; xanthan gum) dissolved in 9.8 wt.% of water was added to obtain the flowable formulation.

Formulation Example 8 (Dry flowable formulation)

Sodium alkylbenzenesulfonate (15 wt.%), polypropylene glycol polyethylene glycol ether (85 wt.%) and $10^{10}$ conidia of *Drechslera* MH-5011 per gram of a dry flowable formulation to be obtained were mixed to obtain the dry flowable formulation.

Formulation Example 9 (Dust)

"Emulgen 910" (trade name; product of Kao Corporation; polyoxyethylenenonyl phenyl ether; 0.5 wt.%) and kaolin clay (99.5 wt.%) were thoroughly ground and mixed, followed by the addition of $10^8$ conidia of *Drechslera* MH-9011 per gram of a dust to be obtained. The dust was hence obtained.

Formulation Example 10 (Emulsion)

In a mixture of 5 wt.% of lecithin and 94 wt.% of heavy white oil, $10^{10}$ conidia of *Drechslera* MH-5511 were suspended per gram of an emulsion to be formulated. An equiamount of 1 wt.% Triton X-100 was added to the suspension. The resultant mixture was mixed and emulsified to obtain the emulsion.

Formulation Example 11 (Granular formulation)

After 9 wt.% of the finely ground chemical herbicide, CNP (Herbicide A in tables), 2 wt.% of "Neopelex" (trade mark; product of Kao Corporation), 2 wt.% of "Sun Ekis P252" (trade name; product of Sanyo-Kokusaku Pulp Co., Ltd.) and 87 wt.% of zeolite were thoroughly mixed, a spore suspension containing $10^8$ conidia of *Drechslera* MH-5018 per gram of a granular formulation to be formulated was added to the resultant mixture to moisten the same. The thus-moistened mass was then extruded into green granules by a small extruder. The green granules were dried in air, crushed and then processed by a shifting machine, whereby granules of 0.3-2 mm were obtained.

Formulation Example 12 (Granular formulation)

After 3.5 wt.% of the chemical herbicide, butachlor (Herbicide H in tables), 2 wt.% of "Neopelex" (trade mark; product of Kao Corporation), 2 wt.% of "Sun Ekis P252" (trade name; product of Sanyo-Kokusaku Pulp Co., Ltd.) and 92.5 wt.% of zeolite were thoroughly mixed, a spore suspension containing $10^8$ conidia of MH-5511 per gram of a granular formulation to be formulated was added to the resultant mixture to moisten the same. The thus-moistened mass was then extruded into green granules by a small extruder. The green granules were dried in air, crushed and then processed by a shifting machine, whereby granules of 0.3-2 mm were obtained.

16

Formulation Example 13 (Granular formulation)

After 4 wt.% of the finely ground chemical herbicide, mefenasate (Herbicide E in tables), 2 wt.% of "Neopelex" (trade mark; product of Kao Corporation), 2 wt.% of "Sun Ekis P252" (trade name; product of Sanyo-Kokusaku Pulp Co., Ltd.) and 92 wt.% of zeolite were thoroughly mixed, a spore suspension containing $10^8$ conidia of *Drechslera* MH-5511 per gram of a granular formulation to be formulated was added to the resultant mixture to moisten the same. The thus-moistened mass was then extruded into green granules by a small extruder. The green granules were dried in air, crushed and then processed by a shifting machine, whereby granules of 0.3-2 mm were obtained.

Formulation Example 14 (Granular formulation)

After 0.2 wt.% of the finely ground chemical herbicide, bensulfuron (Herbicide P in tables), 2 wt.% of "Neopelex" (trade mark; product of Kao Corporation), 2 wt.% of "Sun Ekis P252" (trade name; product of Sanyo-Kokusaku Pulp Co., Ltd.) and 95.8 wt.% of zeolite were thoroughly mixed, a spore suspension containing $10^8$ conidia of *Drechslera* MH-5511 per gram of a granular formulation to be formulated was added to the resultant mixture to moisten the same. The thus-moistened mass was then extruded into green granules by a small extruder. The green granules were dried in air, crushed and then processed by a shifting machine, whereby granules of 0.3-2 mm were obtained.

Formulation Example 15 (Granular formulation)

After 0.3 wt.% of the finely ground chemical herbicide, pyrazosulfron ethyl (Herbicide Q in tables), 2 wt.% of "Gosenol GL-05s" (product of The Nippon Synthetic Chemical Industry Co., Ltd.; PVA), 2 wt.% of "Sun Ekis P252" (trade name; product of Sanyo-Kokusaku Pulp Co., Ltd.; sodium lignin sulfonate) and 95.7 wt.% of clay were thoroughly mixed, a spore suspension containing $10^8$ conidia of *Drechslera* MH-9011 per gram of a granular formulation to be formulated was added to the resultant mixture to moisten the same. The thus-moistened mass was then extruded into green granules by a small extruder. The green granules were dried in air, crushed and then processed by a shifting machine, whereby granules of 0.3-1 mm were obtained.

Formulation Example 16 (Granular formulation)

After 0.3 wt.% of the finely ground chemical herbicide, oxadiazon (Herbicide D in tables), 2 wt.% of "Gosenol GL-05s" (product of The Nippon Synthetic Chemical Industry Co., Ltd.; PVA), 2 wt.% of "Sun Ekis P252" (product of Sanyo-Kokusaku Pulp Co., Ltd.; sodium lignin sulfonate) and 94.5 wt.% of clay were thoroughly mixed, a spore suspension containing $10^8$ conidia of *Drechslera* MH-4418 per gram of a granular formulation to be formulated was added to the resultant mixture to moisten the same. The thus-moistened mass was then extruded into green granules by a small extruder. The green granules were dried in air, crushed and then processed by a shifting machine, whereby granules of 0.3-1 mm were obtained.

Formulation Example 17 (Granular formulation)

After 1.5 wt.% of the finely ground chemical herbicide, simetryne (Herbicide S in tables), 2 wt.% of "Gosenol GL-05s" (product of The Nippon Synthetic Chemical Industry Co., Ltd.; PVA), 2 wt.% of "Sun Ekis P252" (product of Sanyo-Kokusaku Pulp Co., Ltd.; sodium lignin sulfonate) and 94.5 wt.% of clay were thoroughly mixed, a spore suspension containing $10^8$ conidia of *Drechslera* MH-1889 per gram of a granular formulation to be formulated was added to the resultant mixture to moisten the same. The thus-moistened mass was then extruded into green granules by a small extruder. The green granules were dried in air, crushed and then processed by a shifting machine, whereby granules of 0.3-1 mm were obtained.

Formulation Example 18 (Granular formulation)

After 6 wt.% of the chemical herbicide, molinate (Herbicide N in tables), 2 wt.% of "Neopelex" (trade mark; product of Kao Corporation), 2 wt.% of "Sun Ekis P252" (product of Sanyo-Kokusaku Pulp Co., Ltd.; sodium lignin sulfonate) and 90 wt.% of zeolite were thoroughly mixed, a spore suspension containing $10^8$ conidia of *Drechslera* MH-5017 per gram of a granular formulation to be formulated was added to the resultant mixture to moisten the same. The thus-moistened mass was then extruded into green granules by

17

a small extruder. The green granules were dried in air, crushed and then processed by a shifting machine, whereby granules of 0.3-2 mm were obtained.

Formulation Example 19 (Wettable powder)

A mixture of 30 wt.% of the chemical herbicide, chlormethoxynil (Herbicide B in tables), 2 wt.% of "Neopelex" (trade mark; product of Kao Corporation), 2 wt.% of Triton (X-100) and 5 wt.% of white carbon was impregnated with a spore suspension containing $10^9$ conidia of *Drechslera* MH-4415 per gram of a wettable powder to be formulated. Air-dried diatomaceous earth (61 wt.%) was then added to the mixture so impregnated. They were then thoroughly ground to obtain the wettable powder.

Formulation Example 20 (Wettable powder)

A mixture of 40 wt.% of the chemical herbicide, propanil (Herbicide J in tables), 2 wt. % of "Neopelex" (trade mark; product of Kao Corporation), 2 wt.% of Triton (X-100) and 5 wt.% of white carbon was impregnated with a spore suspension containing $10^9$ conidia of *Drechslera* MH-5511 per gram of a wettable powder to be formulated. Air-dried diatomaceous earth (51 wt.%) was then added to the mixture so impregnated. They were then thoroughly ground to obtain the wettable powder.

Formulation Example 21 (Wettable powder)

A mixture of 20 wt.% of the chemical herbicide, benthiocarb (Herbicide L in tables), 2 wt.% of "Neopelex" (trade mark; product of Kao Corporation), 2 wt.% of Triton (X-100) and 5 wt.% of white carbon was impregnated with a spore suspension containing $10^9$ conidia of *Drechslera* MH-5018 per gram of a wettable powder to be formulated. Air-dried diatomaceous earth (71 wt.%) was then added to the mixture so impregnated. They were then thoroughly ground to obtain the wettable powder.

Formulation Example 22 (Wettable powder)

A mixture of 40 wt.% of the chemical herbicide, naproanilide (Herbicide F in tables), 2 wt.% of "Neopelex" (trade mark; product of Kao Corporation; sodium dodecylbenzenesulfonate), 1 wt.% of "Neugen EA 80" (trade name, product of Sanyo Chemical Industries, Ltd.; polyoxyethylenenonylphenyl ether), 5 wt.% of white carbon and 52 wt.% of diatomaceous earth was impregnated with a spore suspension containing $10^9$ conidia of *Drechslera* MH-2653 per gram of a wettable powder to be formulated, followed by thorough mixing and grinding to obtain the wettable powder.

Formulation Example 23 (Wettable powder)

A mixture of 20 wt.% of the chemical herbicide dimepiperate (Herbicide M in tables), 2 wt.% of sodium alkylbenzenesulfonate, 1 wt.% of polyoxyethylene alkylphenylether and 77 wt.% of zeaklite was impregnated with a spore suspension containing $10^9$ conidia of *Drechslera* MH-0122 per gram of a wettable powder to be formulated, followed by thorough mixing and grinding to obtain the wettable powder.

Formulation Example 24 (Wettable powder)

A mixture of 40 wt.% of the chemical herbicide clomeprop (Herbicide K in tables), 5 wt.% of white carbon, 6 wt.% of polyoxyethylene alkylphenylether ammonium sulfate salt, 2 wt.% of sodium lignin sulfonate and 47 wt.% of diatomaceous earth was thoroughly mixed and ground by a Jet-O-Miser, followed by impregnation with a spore suspension containing $10^9$ conidia of *Drechslera* MH-0015 per gram of a wettable powder to be formulated. The resultant mixture was thoroughly mixed and ground to obtain the wettable powder.

Formulation Example 25 (Flowable formulation)

After 45 wt.% of the finely ground chemical herbicide, CNP (Herbicide A in tables) and 10 wt.% of "Sun Ekis P252" dissolved in 35 wt.% of water were mixed and wet-ground, 0.4 wt.% of "Kelzan S" (trade name: product of Kelco Corp.) dissolved in 9.6 wt.% of a spore suspension containing $10^9$ conidia of *Drechslera* MH-0042 per gram of a flowable formulation to be formulated was added to the resultant mixture. The thus-

18

obtained mixture was then mixed to obtain the flowable formulation.

Formulation Example 26 (Flowable formulation)

After 10 wt.% of the chemical herbicide, pretilachlor (Herbicide I in tables) and 10 wt.% of "Sun Ekis P252" dissolved in 70 wt.% of water were mixed and wet-ground, 0.4 wt.% of "Kelzan S" (trade name: product of Kelco Corp.) dissolved in 9.6 wt.% of a spore suspension containing $10^9$ conidia of *Drechslera* MH-5511 per gram of a flowable formulation to be formulated was added to the resultant mixture. The thus-obtained mixture was then mixed to obtain the flowable formulation.

Formulation Example 27 (Flowable formulation)

After 56.7 wt.% of water was added to the mixture of 30 wt.% of the chemical herbicide, benthiocarb (Herbicide L in tables), 2 wt.% of sodium lignin sulfonate; 0.3 wt.% of xanthan gum and 1 wt.% of polyoxyethylene alkylarylether, 10 wt.% of a spore suspension containing $10^9$ conidia of *Drechslera* MH-5017 per gram of a flowable formulation to be formulated was added to the resultant mixture. The thus-obtained mixture was then finely ground by a sand grinder to obtain the flowable formulation.

Formulation Example 28 (Flowable formulation)

After 30 wt.% of the chemical herbicide, esprocarb (Herbicide O in tables), 10 wt.% of "Sun Ekis P252" (trade name; described above) dissolved in 40 wt.% of water and 10 wt.% of a spore suspension containing $10^9$ conidia of *Drechslera* MH-5011 per gram of a flowable formulation to be formulated were mixed, the resultant mixture was mixed and wet-ground. Then 0.4 wt.% of "Kelzan S" (trade name: product of Kelco Corp.; xanthan gum) dissolved in 9.6 wt.% of water was added to the mixture to obtain the flowable formulation.

Formulation Example 29 (Dry flowable formulation)

The finely-ground chemical herbicide, pyrazolate (Herbicide R in tables; 60 wt.%), sodium alkylbenzenesulfonate (5 wt.%), polypropylene glycol polyethylene glycol ether (35 wt.%) and $10^9$ conidia of *Drechslera* MH-2781 per gram of the chemical herbicide were mixed to obtain the dry flowable formulation.

Formulation Example 30 (Dust)

The finely-ground chemical herbicide, bensulfuron (Herbicide P in tables; 0.2 wt.%), "Emulgen 910" (trade name; product of Kao Corporation; polyoxyethylenenonyl phenyl ether; 0.5 wt.%) and kaolin clay (99.3 wt.%) were thoroughly mixed and ground, followed by the addition of $10^8$ conidia of *Drechslera* MH-9011 per gram of a dust to be obtained. The dust was hence obtained.

Formulation Example 31 (Dust)

The finely-ground chemical herbicide, mefenacet (Herbicide E in tables; 4 wt.%), "Emulgen 910" (trade name; product of Kao Corporation; polyoxyethylenenonyl phenyl ether; 1 wt.%, sodium lignin sulfonate (3 wt.%), polyoxyethylene alkylarylether (2 wt.%) and kaolin clay (90 wt.%) were mixed and ground, followed by the addition of $10^9$ conidia of *Drechslera* MH-2883 per gram of a dust to be obtained. The dust was hence obtained.

Formulation Example 32 (Emulsion)

In a mixture of 1 wt.% of the chemical herbicide, bensulfuron (Herbicide P in tables), 5 wt.% of lecithin and 94 wt.% of heavy white oil, $10^9$ conidia of *Drechslera* MH-5511 were suspended per gram of an emulsion to be formulated. An equiamount of 1 wt.% Triton X-100 was added to the suspension. The resultant mixture was mixed and emulsified to obtain the emulsion.

Formulation Example 33 (Emulsion)

In a mixture of 2 wt.% of the chemical herbicide, biphenox (Herbicide C in tables), 5 wt.% of lecithin and 92 wt.% of heavy white oil, $10^{10}$ conidia of *Drechslera* MH-0060 were suspended per gram of an emulsion to be formulated. An equiamount of 1 wt.% Triton X-100 was added to the suspension. The resultant mixture was mixed and emulsified to obtain the emulsion.

In addition to the above formulation examples, formulation was conducted with respect to the combinations of conidia of various strains of *Drechslera spp.* and various chemical herbicides. It is therefore to be noted that formulations are not limited to those exemplified above.

Example 1

Herbicidal Effects of Weed Control Compositions, Which Contain *Drechslera* Strains and Chemical Herbicides in Combination, against Barnyard Grass - Granular Formulations

Barnyard grass seeds were planted in 1/1000-are pots contained in lowland soil, and the plants raising from the seeds were reared to the 1.5 leaf stage. The plants were irrigated to keep about 3 cm depth in water and treated with 30 mg (equivalent to one tenth of the standard dosage) of the granular formulations prepared in Formulation Examples 11-18, respectively. The plants were then allowed to grow in a green house which was maintained at 35°C during the day time and at 20°C at night. Twenty days after the treatment, the remaining populations in the individual pots were counted. The control values against barnyard grass were calculated in accordance with the following formula and are shown in Tables 6-1 to 6-3.

$$\text{Control value} = \frac{\text{Remaining population in untreated group} - \text{Remaining population in treated group}}{\text{Remaining population in untreated group}} \times 100$$

In the tables, letters A-O represent the following herbicides.

A:  CNP
B:  Chlormethoxynil
C:  Bifenox
D:  Oxadiazon
E:  Mefenasate
F:  Naproanilide
G:  NSK-850
H:  Butachlor
I:  Pretilachlor
J:  Propanil
K:  Clomeprop
L:  Benthiocarb
M:  Dimepiperate
N:  Molinate
O:  Esprocarb

20

Table 6-1

| Herbicidal Effects of *Drechslera spp.*-Diphenyl Ether Chemical Herbicide Compositions | | | | | |
|---|---|---|---|---|---|
| Microorganism | Chemical herbicide | | | | |
| | - | A | B | C | D |
| - | 0 | 10 | 25 | 20 | 40 |
| MH-0015 | 10 | 100 | 100 | 100 | 100 |
| MH-0042 | 5 | 100 | 100 | 100 | 100 |
| MH-0060 | 5 | 100 | 100 | 100 | 100 |
| MH-0122 | 5 | 100 | 100 | 100 | 100 |
| MH-1889 | | 100 | 100 | 100 | 100 |
| MH-2653 | 15 | 100 | 100 | 100 | 100 |
| MH-2679 | 10 | 100 | 100 | 100 | 100 |
| MH-2781 | 5 | 100 | 100 | 100 | 100 |
| MH-2883 | 5 | 100 | 100 | 100 | 100 |
| MH-2895 | 5 | 100 | 100 | 100 | 100 |
| MH-2990 | 10 | 100 | 100 | 100 | 100 |
| MH-2998 | 5 | 100 | 100 | 100 | 100 |
| MH-4415 | 10 | 100 | 100 | 100 | 100 |
| MH-4418 | 10 | 100 | 100 | 100 | 100 |
| MH-5011 | 5 | 100 | 100 | 100 | 100 |
| MH-5017 | 10 | 100 | 100 | 100 | 100 |
| MH-5018 | 10 | 100 | 100 | 100 | 100 |
| MH-5511 | 10 | 100 | 100 | 100 | 100 |
| MH-9011 | 10 | 100 | 100 | 100 | 100 |

Table 6-2

| Herbicidal Effects of *Drechslera spp.*-Acetanilide Chemical Herbicide Compositions | | | | | | | | |
|---|---|---|---|---|---|---|---|---|
| Microorganism | Chemical herbicide | | | | | | | |
| | - | E | F | G | H | I | J | K |
| - | 0 | 35 | 5 | 50 | 30 | 40 | 10 | 25 |
| MH-0015 | 5 | 100 | 90 | 100 | 100 | 100 | 85 | 100 |
| MH-0042 | 5 | 100 | 85 | 100 | 100 | 100 | 90 | 100 |
| MH-0060 | 5 | 100 | 85 | 100 | 100 | 100 | 85 | 100 |
| MH-0122 | 5 | 100 | 85 | 100 | 100 | 100 | 90 | 100 |
| MH-1889 | 5 | 100 | 90 | 100 | 100 | 100 | 95 | 100 |
| MH-2653 | 15 | 100 | 95 | 100 | 100 | 100 | 90 | 100 |
| MH-2679 | 10 | 100 | 90 | 100 | 100 | 100 | 85 | 100 |
| MH-2781 | 5 | 100 | 85 | 100 | 100 | 100 | 85 | 100 |
| MH-2883 | 5 | 100 | 85 | 100 | 100 | 100 | 85 | 100 |
| MH-2895 | 5 | 100 | 85 | 100 | 100 | 100 | 80 | 100 |
| MH-2990 | 10 | 100 | 90 | 100 | 100 | 100 | 90 | 100 |
| MH-2998 | 5 | 100 | 85 | 100 | 100 | 100 | 90 | 100 |
| MH-4415 | 10 | 100 | 100 | 100 | 100 | 100 | 100 | 100 |
| MH-4418 | 5 | 100 | 100 | 100 | 100 | 100 | 100 | 100 |
| MH-5011 | 5 | 100 | 100 | 100 | 100 | 100 | 100 | 100 |
| MH-5017 | 10 | 100 | 100 | 100 | 100 | 100 | 100 | 100 |
| MH-5018 | 10 | 100 | 100 | 100 | 100 | 100 | 100 | 100 |
| MH-5511 | 10 | 100 | 100 | 100 | 100 | 100 | 100 | 100 |
| MH-9011 | 10 | 100 | 100 | 100 | 100 | 100 | 100 | 100 |

Table 6-3

| Herbicidal Effects of *Drechslera spp.*-Thiolcarbamate Chemical Herbicide Compositions | | | | | |
|---|---|---|---|---|---|
| Microorganism | Chemical herbicide | | | | |
| | - | L | M | N | O |
| - | 0 | 10 | 10 | 10 | 20 |
| MH-0015 | 10 | 90 | 95 | 85 | 90 |
| MH-0042 | 5 | 85 | 85 | 90 | 80 |
| MH-0060 | 5 | 85 | 80 | 90 | 90 |
| MH-0122 | 5 | 85 | 90 | 85 | 80 |
| MH-1889 | 5 | 95 | 90 | 90 | 95 |
| MH-2653 | 15 | 95 | 95 | 90 | 90 |
| MH-2679 | 10 | 90 | 90 | 85 | 90 |
| MH-2781 | 5 | 85 | 80 | 90 | 90 |
| MH-2883 | 5 | 85 | 85 | 85 | 85 |
| MH-2895 | 5 | 85 | 90 | 85 | 90 |
| MH-2990 | 10 | 90 | 90 | 95 | 85 |
| MH-2998 | 5 | 85 | 85 | 80 | 85 |
| MH-4415 | 5 | 100 | 100 | 100 | 100 |
| MH-4418 | 10 | 100 | 100 | 100 | 100 |
| MH-5011 | 5 | 100 | 100 | 100 | 100 |
| MH-5017 | 10 | 100 | 100 | 100 | 100 |
| MH-5018 | 10 | 100 | 100 | 100 | 100 |
| MH-5511 | 10 | 100 | 100 | 100 | 100 |
| MH-9011 | 10 | 100 | 100 | 100 | 100 |

It is appreciated from Table 6-1 that, in the case of the compositions containing the *Drechslera* strains according to the present invention in combination with the diphenyl ether herbicides, i.e., CNP, chlormethoxynil and bifenox or the diazine herbicide, i.e., oxadiazon, respectively, the herbicidal activities were synergistically enhanced compared with their single application.

It is understood from Table 6-2 that, in the case of the compositions containing the *Drechslera* strains according to the present invention in combination with the anilide herbicides, i.e., mefenacet, naproanilide, NSK-850, butachlor, pretilachlor, propanil and clomeprop, respectively, the herbicidal activities were synergistically enhanced compared with their single application.

It is envisaged from Table 6-3 that, in the case of the compositions containing the *Drechslera* strains according to the present invention in combination with the thiolcarbamate herbicides, i.e., benthiocarb, dimepiperate, molinate and esprocarb, respectively, the herbicidal activities were synergistically enhanced compared with their single application.

From the foregoing results, outstanding synergistic effects can be observed from compositions which contain *Drechslera* strains having pathogenecity against barnyard grass in combination with diphenyl ether herbicides, anilide herbicides, thiolcarbamate herbicides, diazine herbicides and like herbicides, said herbicides having all been employed as barnyard grass herbicides. Consequently, it has become possible to satisfactorily control barnyard grass by using such chemical herbicides even at a dosage as low as one tenth to one hundredth of their conventional dosage.

Example 2

Herbicidal Effects of Weed Control Compositions, Which Contain *Drechslera* Strains and Chemical Herbicides in Combination, against Barnyard Grass - Flowable Formulations

Barnyard grass seeds were planted in 1/1000-are pots contained in lowland soil, and the plants raising from the seeds were reared to the 1.5 leaf stage. The plants were irrigated to keep about 3 cm depth in water and treated with 1 $\mu\ell$ (equivalent to one tenth of the standard dosage) of the flowable formulations prepared in the above formulation examples, respectively. The plants were then allowed to grow in a green house which was maintained at 35°C during the day time and at 20°C at night. Twenty days after the treatment, the remaining populations in the individual pots were counted. The control values against

22

barnyard grass were calculated in accordance with the following formula and are shown in Tables 7-1 to 7-3.

$$\text{Control value} = \frac{\text{Remaining population in untreated group} - \text{Remaining population in treated group}}{\text{Remaining population in untreated group}} \times 100$$

It is appreciated from Table 7-1 that, in the case of the compositions containing the *Drechslera* strains according to the present invention in combination with the diphenyl ether herbicide, i.e., CNP, respectively, the herbicidal activities were synergistically enhanced compared with their single application.

It is understood from Table 7-2 that, in the case of the compositions containing the *Drechslera* strains according to the present invention in combination with the anilide herbicides, i.e., mefenacet and pretilachlor, respectively, the herbicidal activities were synergistically enhanced compared with their single application.

It is envisaged from Table 7-3 that, in the case of the compositions containing the *Drechslera* strains according to the present invention in combination with the thiolcarbamate herbicides, i.e., benthiocarb and molinate, respectively, the herbicidal activities were synergistically enhanced compared with their single application.

Table 7-1

| Herbicidal Effects of *Drechslera spp.*-CNP Compositions | | |
|---|---|---|
| Microorganism | Chemical herbicide | |
| | - | CNP |
| - | 0 | 15 |
| MH-0015 | 5 | 100 |
| MH-0042 | 5 | 100 |
| MH-0060 | 5 | 100 |
| MH-0122 | 5 | 100 |
| MH-1889 | 10 | 100 |
| MH-2653 | 15 | 100 |
| MH-2679 | 10 | 100 |
| MH-2781 | 5 | 100 |
| MH-2883 | 5 | 100 |
| MH-2895 | 5 | 100 |
| MH-2990 | 10 | 100 |
| MH-2998 | 5 | 100 |
| MH-4415 | 5 | 100 |
| MH-4418 | 5 | 100 |
| MH-5011 | 10 | 100 |
| MH-5017 | 5 | 100 |
| MH-5018 | 10 | 100 |
| MH-5511 | 10 | 100 |
| MH-9011 | 10 | 100 |

Table 7-2

| Herbicidal Effects of *Drechslera spp.*-Mefenacet/Pretilachlor Compositions | | | |
|---|---|---|---|
| Microorganism | Chemical herbicide | | |
| | - | Mefenacet | Pretilachlor |
| - | 0 | 25 | 30 |
| MH-0015 | 5 | 100 | 100 |
| MH-0042 | 5 | 100 | 100 |
| MH-0060 | 5 | 100 | 100 |
| MH-0122 | 5 | 100 | 100 |
| MH-1889 | 10 | 100 | 100 |
| MH-2653 | 15 | 100 | 100 |
| MH-2679 | 10 | 100 | 100 |
| MH-2781 | 5 | 100 | 100 |
| MH-2883 | 5 | 100 | 100 |
| MH-2895 | 5 | 100 | 100 |
| MH-2990 | 10 | 100 | 100 |
| MH-2998 | 5 | 100 | 100 |
| MH-4415 | 5 | 100 | 100 |
| MH-4418 | 5 | 100 | 100 |
| MH-5011 | 10 | 100 | 100 |
| MH-5017 | 10 | 100 | 100 |
| MH-5018 | 10 | 100 | 100 |
| MH-5511 | 10 | 100 | 100 |
| MH-9011 | 10 | 100 | 100 |

Table 7-3

| Herbicidal Effects of *Drechslera spp.*-Benthiocarb/Molinate Compositions | | | |
|---|---|---|---|
| Microorganism | Chemical herbicide | | |
| | - | Benthiocarb | Molinate |
| - | 0 | 15 | 10 |
| MH-0015 | 10 | 90 | 85 |
| MH-0042 | 5 | 80 | 80 |
| MH-0060 | 5 | 80 | 85 |
| MH-0122 | 5 | 85 | 85 |
| MH-1889 | 5 | 95 | 95 |
| MH-2653 | 15 | 90 | 95 |
| MH-2679 | 10 | 95 | 90 |
| MH-2781 | 5 | 80 | 80 |
| MH-2883 | 5 | 85 | 85 |
| MH-2895 | 5 | 85 | 80 |
| MH-2990 | 10 | 95 | 90 |
| MH-2998 | 5 | 80 | 85 |
| MH-4415 | 5 | 100 | 100 |
| MH-4418 | 5 | 100 | 100 |
| MH-5011 | 10 | 100 | 100 |
| MH-5017 | 10 | 100 | 100 |
| MH-5018 | 10 | 100 | 100 |
| MH-5511 | 10 | 100 | 100 |
| MH-9011 | 10 | 100 | 100 |

Example 3

Herbicidal Effects of Weed Control Compositions, Which Contain *Drechslera* Strains and Chemical Herbicides in Combination, against Barnyard Grass - Flowable Formulations

Barnyard grass seeds were planted in 1/1000-are pots contained in lowland soil, and the plants raising from the seeds were reared to the 1.5 leaf stage. The plants were irrigated to keep about 3 cm depth in water and treated with 1.5 $\mu\ell$ (equivalent to one tenth of the standard dosage) of the flowable formulations prepared in the above formulation examples, respectively. The plants were then allowed to grow in a green house which was maintained at 35°C during the day time and at 20°C at night. Twenty days after the treatment, the remaining populations in the individual pots were counted. The control values against barnyard grass were calculated in accordance with the following formula and are shown in Table 8.

$$\text{Control value} = \frac{\text{Remaining population in untreated group} - \text{Remaining population in treated group}}{\text{Remaining population in untreated group}} \times 100$$

In the table, A, E, I and L represent the following herbicides.

A:    CNP
E:    Mefenacet
I:    Pretilachlor
L:    Benthiocarb

It is appreciated from Table 8 that, in the case of the compositions containing the *Drechslera* strains according to the present invention in combination with the diphenyl ether herbicide, i.e., CNP, respectively, the herbicidal activities were synergistically enhanced compared with their single application.

It is understood from Table 8 that, in the case of the compositions containing the *Drechslera* strains according to the present invention in combination with the anilide herbicides, i.e., mefenacet and pretilachlor, respectively, the herbicidal activities were synergistically enhanced compared with their single application.

It is envisaged from Table 8 that, in the case of the compositions containing the *Drechslera* strains according to the present invention in combination with the thiol carbamate herbicides, i.e., benthiocarb and molinate, respectively, the herbicidal activities were synergistically enhanced compared with their single application.

Table 8

| Herbicidal Effects of *Drechslera spp.*-Chemical Herbicide Compositions | | | | | |
|---|---|---|---|---|---|
| Microorganism | Chemical herbicide | | | | |
| | - | A | E | I | L |
| - | 0 | 10 | 20 | 30 | 10 |
| MH-0015 | 10 | 90 | 90 | 90 | 90 |
| MH-0042 | 5 | 85 | 85 | 85 | 85 |
| MH-0060 | 5 | 85 | 85 | 85 | 85 |
| MH-0122 | 5 | 85 | 85 | 85 | 85 |
| MH-1889 | 5 | 95 | 95 | 100 | 95 |
| MH-2653 | 15 | 95 | 95 | 95 | 95 |
| MH-2679 | 10 | 90 | 90 | 90 | 90 |
| MH-2781 | 5 | 85 | 85 | 85 | 85 |
| MH-2883 | 5 | 85 | 85 | 85 | 85 |
| MH-2895 | 5 | 85 | 85 | 85 | 85 |
| MH-2990 | 10 | 90 | 90 | 90 | 90 |
| MH-2998 | 5 | 85 | 85 | 85 | 85 |
| MH-4415 | 5 | 100 | 100 | 100 | 100 |
| MH-4418 | 5 | 100 | 100 | 100 | 100 |
| MH-5011 | 10 | 100 | 100 | 100 | 100 |
| MH-5017 | 10 | 100 | 100 | 100 | 100 |
| MH-5018 | 10 | 100 | 100 | 100 | 100 |
| MH-5511 | 10 | 100 | 100 | 100 | 100 |
| MH-9011 | 10 | 100 | 100 | 100 | 100 |

Example 4

Herbicidal Effects of Weed Control Compositions, Which Contained *Drechslera* Strains and Chemical Herbicides in Combination, against Barnyard Grass and Broadleaf Weeds

Barnyard grass, monochoria (*Monochoria vaginalis*) and narrowleaf waterplantain were separately seeded in lowland soil which was contained in 1/1000-are pots, and were reared to the 1.5 leaf stage. The pots which were in a state irrigated to about 3 cm in water depth were treated with 1.5 mg (equivalent to one tenth of the standard dosage) of the various compositions prepared above in Formulation Examples 1-33, respectively. The seedlings were then allowed to grow in a green house which was maintained at 35°C during the day time and at 20°C at night. Twenty days after the treatment, the remaining populations in the individual pots were counted. The control values against barnyard grass, monochoria or narrowleaf waterplantain were calculated in accordance with the following formula and are shown in Tables 9-1 to 9-3.

$$\text{Control value} = \frac{\text{Remaining population in untreated group} - \text{Remaining population in treated group}}{\text{Remaining population in untreated group}} \times 100$$

In the tables, P-S represent the following herbicides.

A: CNP
P: Bensulfuron methyl
Q: Pyrazosulfron ethyl
R: Pyrazolate
S: Simetryne

Table 9-1

| Herbicidal Effects of *Drechslera spp.*-Chemical Herbicide Compositions against Barnyard Grass | | | | | |
|---|---|---|---|---|---|
| Microorganism | Chemical herbicide | | | | |
| | - | P | Q | R | S |
| - | 0 | 10 | 20 | 30 | 10 |
| MH-0015 | 10 | 90 | 90 | 90 | 90 |
| MH-0042 | 5 | 85 | 85 | 85 | 85 |
| MH-0060 | 5 | 85 | 85 | 85 | 85 |
| MH-0122 | 5 | 85 | 85 | 85 | 85 |
| MH-1889 | 5 | 95 | 90 | 100 | 85 |
| MH-2653 | 15 | 95 | 95 | 95 | 95 |
| MH-2679 | 10 | 90 | 90 | 90 | 90 |
| MH-2781 | 5 | 85 | 85 | 85 | 85 |
| MH-2883 | 5 | 85 | 85 | 85 | 85 |
| MH-2895 | 5 | 85 | 85 | 85 | 85 |
| MH-2990 | 10 | 90 | 90 | 90 | 90 |
| MH-2998 | 5 | 85 | 85 | 85 | 85 |
| MH-4415 | 5 | 100 | 100 | 100 | 100 |
| MH-4418 | 5 | 100 | 100 | 100 | 100 |
| MH-5011 | 10 | 100 | 100 | 100 | 100 |
| MH-5017 | 10 | 100 | 100 | 100 | 100 |
| MH-5018 | 10 | 100 | 100 | 100 | 100 |
| MH-5511 | 10 | 100 | 100 | 100 | 100 |
| MH-9011 | 10 | 100 | 100 | 100 | 100 |

Table 9-2

| Herbicidal Effects of *Drechslera spp.*-Chemical Herbicide Compositions against Monochoria | | | | | |
|---|---|---|---|---|---|
| Microorganism | Chemical herbicide | | | | |
| | - | P | Q | R | S |
| - | 0 | 90 | 90 | 80 | 80 |
| MH-0015 | 0 | 90 | 90 | 90 | 90 |
| MH-0042 | 0 | 85 | 85 | 85 | 85 |
| MH-0060 | 0 | 85 | 85 | 85 | 85 |
| MH-0122 | 0 | 85 | 85 | 85 | 85 |
| MH-1889 | 0 | 90 | 95 | 90 | 90 |
| MH-2653 | 0 | 95 | 95 | 95 | 95 |
| MH-2679 | 0 | 90 | 90 | 90 | 90 |
| MH-2781 | 0 | 85 | 85 | 85 | 85 |
| MH-2883 | 0 | 85 | 85 | 85 | 85 |
| MH-2895 | 0 | 85 | 85 | 85 | 85 |
| MH-2990 | 0 | 90 | 90 | 90 | 90 |
| MH-2998 | 0 | 85 | 85 | 85 | 85 |
| MH-4415 | 0 | 100 | 100 | 100 | 100 |
| MH-4418 | 0 | 100 | 100 | 100 | 100 |
| MH-5011 | 0 | 100 | 100 | 100 | 100 |
| MH-5017 | 0 | 100 | 100 | 100 | 100 |
| MH-5018 | 0 | 100 | 100 | 100 | 100 |
| MH-5511 | 0 | 100 | 100 | 100 | 100 |
| MH-9011 | 0 | 100 | 100 | 100 | 100 |

Table 9-3

| Herbicidal Effects of *Drechslera spp.*-Chemical Herbicide Compositions Against Narrowleaf Waterplantain | | | | | |
|---|---|---|---|---|---|
| Microorganism | Chemical herbicide | | | | |
| | - | P | Q | R | S |
| - | 0 | 85 | 85 | 80 | 90 |
| MH-0015 | 0 | 90 | 90 | 90 | 90 |
| MH-0042 | 0 | 85 | 85 | 85 | 85 |
| MH-0060 | 0 | 85 | 85 | 85 | 85 |
| MH-0122 | 0 | 85 | 85 | 85 | 85 |
| MH-1889 | 0 | 95 | 95 | 100 | 95 |
| MH-2653 | 0 | 95 | 95 | 95 | 95 |
| MH-2679 | 0 | 90 | 90 | 90 | 90 |
| MH-2781 | 0 | 85 | 85 | 85 | 85 |
| MH-2883 | 0 | 85 | 85 | 85 | 85 |
| MH-2895 | 0 | 85 | 85 | 85 | 85 |
| MH-2990 | 0 | 90 | 90 | 90 | 90 |
| MH-2998 | 0 | 85 | 85 | 85 | 85 |
| MH-4415 | 0 | 100 | 100 | 100 | 100 |
| MH-4418 | 0 | 100 | 100 | 100 | 100 |
| MH-5011 | 0 | 100 | 100 | 100 | 100 |
| MH-5017 | 0 | 100 | 100 | 100 | 100 |
| MH-5018 | 0 | 100 | 100 | 100 | 100 |
| MH-5511 | 0 | 100 | 100 | 100 | 100 |
| MH-9011 | 0 | 100 | 100 | 100 | 100 |

It is appreciated from Table 9-1 that, in the case of the compositions containing the *Drechslera* strains according to the present invention in combination with the sulfonylurea herbicides, i.e., bensulfuron methyl and pyrazosulfron ethyl, the diazine herbicide, i.e., pyrazolate or the triazine herbicide, i.e., simetryne, respectively, the herbicidal activities against barnyard grass were synergistically enhanced compared with their single application.

It is understood from Tables 9-2 and 9-3 that, in the case of the compositions containing the *Drechslera* strains according to the present invention in combination with the sulfonylurea herbicides, i.e., bensulfuron methyl and pyrazosulfron ethyl, the diazine herbicide, i.e., pyrazolate or the triazine herbicide, i.e., simetryne, respectively, the herbicidal activities against the broadleaf weeds, namely, monochoria or narrowleaf waterplantain were enhanced to some extent compared with their single application.

It is also envisaged from Table 9-1 that, in the case of the compositions containing the *Drechslera* strains according to the present invention in combination with the sulfonylurea herbicides, i.e., bensulfuron methyl and pyrazosulfron ethyl, the diazine herbicide, i.e., pyrazolate or the triazine herbicide, i.e., simetryne, respectively, sufficient weed control effects were also exhibited against the broadleaf weeds, namely, monochoria or narrowleaf waterplantain compared with their single application and increased practical utility was observed.

From the foregoing results, it is understood that compositions containing *Drechslera* strains having pathogenecity against barnyard grass in combination with sulfonylurea herbicides such as bensulfuron methyl and pyrazosulfron ethyl, diazine herbicides such as pyrazolate or triazine herbicides such as simetryne - said herbicides having been employed against broadleaf weeds - can exhibit enhanced weed control effects against barnyard grass and also a broadened spectrum and enhanced herbicidal effects against broadleaf weeds although the enhancement of their herbicidal effects against broadleaf weeds is not substantial. These compositions are therefore believed to have practical utility.

The combined use with broadleaf weed herbicides makes it possible to reduce the dosage of conidia of each strain of *Drechslera spp.*. This in turn makes it possible to reduce the production cost.

Example 5

Dosage Reduction of Conidia by Synergistic Effects of *Drechslera* Strains and Chemical Herbicides

Barnyard grass seeds were planted in 1/1000-are pots contained in lowland soil, and the plants raising from the seeds were reared to the 1.5 leaf stage. The plants were irrigated to keep about 3 cm depth in water and treated with predetermined amounts of conidia and also with a prescribed amount (equivalent to one tenth of the standard dosage) of the broadleaf weed herbicide, bensulfuron. The plants were then allowed to grow in a green house which was maintained at 35°C during the day time and at 20°C at night. Twenty days after the treatment, the remaining populations in the individual pots were counted. The control values against barnyard grass were calculated in accordance with the following formula and are shown in Table 10.

$$\text{Control value} = \frac{\text{Remaining population in untreated group} - \text{Remaining population in treated group}}{\text{Remaining population in untreated group}} \times 100$$

Table 10

| Reduction in Conidium Dosage owing to Synergistic Effects of *Drechslera* Strains and Chemical Herbicide | | |
|---|---|---|
| Microorganism MH-5511 | Microorganism alone | Bensulfuron + Microorganism |
| $1 \times 10^6$ | 100 | 100 |
| $3 \times 10^5$ | 80 | 100 |
| $1 \times 10^5$ | 50 | 100 |
| $3 \times 10^4$ | 30 | 70 |
| $1 \times 10^4$ | 10 | 50 |
| 0 | 0 (Untreated) | 10 (Herbicide alone) |

| List of Deposited Microorganisms (International Deposit under the Budapest Treaty) | | |
|---|---|---|
| Name of Strain | Date of Deposit | Deposit No. |
| MH-0015 | June 17, 1989 | FERM BP-2652 |
| MH-0042 | October 28, 1988 | FERM BP-2659 |
| MH-0060 | September 28, 1989 | FERM BP-2657 |
| MH-0122 | August 31, 1989 | FERM BP-2655 |
| MH-1889 | February 7, 1991 | FERM BP-3410 |
| MH-2653 | June 17, 1989 | FERM BP-2653 |
| MH-2679 | August 31, 1989 | FERM BP-2656 |
| MH-2781 | June 8, 1990 | FERM BP-3407 |
| MH-2883 | June 8, 1990 | FERM BP-3408 |
| MH-2895 | June 8, 1990 | FERM BP-3409 |
| MH-4415 | February 22, 1991 | FERM BP-3413 |
| MH-4418 | February 22, 1991 | FERM BP-3414 |
| MH-5011 | February 22, 1991 | FERM BP-3415 |
| MH-5017 | February 7, 1991 | FERM BP-3411 |
| MH-5018 | February 7, 1991 | FERM BP-3412 |
| MH-5511 | March 27, 1991 | FERM BP-3417 |
| MH-9011 | March 27, 1991 | FERM BP-3416 |

## Claims

1. A weed control composition comprising a strain of *Drechslera spp.*, a chemical herbicide and an agriculturally acceptable carrier, said strain of *Drechslera spp.* being free of pathogenicity against economic crops but having pathogenicity against *Echinochloa spp.*

2. The weed control composition of claim 1, wherein the *Drechslera spp.* is *Drechslera monoceras.*

3. The weed control composition of claim 2, wherein the strain of *Drechslera monoceras* is MH-0015 (FERM BP-2652), MH-2653 (FERM BP-2653), MH-2679 (FERM BP-2656), MH-1889 (FERM BP-3410), MH-4415 (FERM BP-3413), MH-4418 (FERM BP-3414), MH-5011 (FERM BP-3415), MH-5017 (FERM BP-3411), MH-5018 (FERM BP-3412), MH-5511 (FERM BP-3417) or MH-9011 (FERM BP-3416).

4. The weed control composition of claim 1, wherein the *Drechslera spp.* is *Drechslera ravenelii.*

5. The weed control composition of claim 4, wherein the strain of *Drechslera ravenelii* is MH-0042 (FERM BP-2659), MH-0060 (FERM BP-2657) or MH-2883 (FERM BP-3408).

6. The weed control composition of claim 1, wherein the *Drechslera spp.* is *Drechslera poae.*

7. The weed control composition of claim 6, wherein the strain of *Drechslera poae* is MH-0122 (FERM BP-2655), MH-2781 (FERM BP-3407) or MH-2895 (FERM BP-3409).

8. The weed control composition of claim 1, wherein the chemical herbicide is a diphenyl ether herbicide.

9. The weed control composition of claim 8, wherein the diphenyl ether herbicide is CNP (chemical name: 2,4,6-trichlorophenyl-4-nitrophenyl ether), chlormethoxynil (chemical name: 2,4-dichlorophenyl-4-nitro-3-methoxhenyl ether) or bifenox (chemical name: methyl 5-(2,4-dichlorophenyl)-2-nitrobenzoate).

10. The weed control composition of claim 1, wherein the chemical herbicide is an anilide herbicide.

11. The weed control composition of claim 10, wherein the anilide herbicide is mefenacet (chemical name: 2-benzothiazol-3-yloxy-N-methylacetanilide), pretilachlor (chemical name: 2-chloro-2',6'-diethyl-N-(n-propoxyethyl)acetanilide), naproanilide (chemical name: 2-(2-naphthoxy)propionic anilide), NSK-850 (chemical name: 2-chloro-N-(3-methoxy-2-theinyl)methyl-2,6-dimethylanilide), butachlor (chemical name: 2-chloro-2',6'-diethyl-N-(butoxymethyl)acetanilide), propanil (chemical name: 3',4'-dichloropropionanilide), or clomeprop (chemical name: 2-(2,4-dichloro-3-methylphenoxy)propionanilide).

12. The weed control composition of claim 1, wherein the chemical herbicide is a thiolcarbamate herbicide.

13. The weed control composition of claim 12, wherein the thiol carbamate herbicide is benthiocarb (chemical name: S-(4-chlorobenzyl)-N,N-diethylthiol carbamate), dimepiperate (chemical name: S-($\alpha$,$\alpha$-dimethylbenzyl)-piperidine-1-carbathioate), molinate (chemical name: S-ethyl-N,N-hexamethylenethiolcarbamate), or esprocarb (chemical name: S-benzyl-N-ethyl-N-(1,2-dimethylpropyl)thiolcarbamate).

14. The weed control composition of claim 1, wherein the chemical herbicide is a diazine herbicide.

15. The weed control composition of claim 14, wherein the diazine herbicide is oxadiazon (chemical name: 5-tert-butyl-3-(2,4-dichloro-5-isopropoxyphenyl)-1,3,4-oxazol-2(3H)-one), or pyrazolate (chemical name: 4-(2,4-dichlorobenzoyl)-1,3-dimethyl-1H-pyrasol-5-yl-p-toluenesulfonate).

16. The weed control composition of claim 1, wherein the chemical herbicide is a sulfonylurea herbicide.

17. The weed control composition of claim 16, wherein the sulfonylurea herbicide is bensulfuron methyl (chemical name: methyl 2-[3-(4,6-dimethoxypyrimidin-2-yl)ureidosulfonylmethyl]benzoate) or pyrazosulfuron ethyl (chemical name: ethyl 5-[3-(4,6-dimethoxypyrimidin-2-yl)ureidosulfonyl]-1-methylpyrazole-4-carboxylate).

18. The weed control composition of claim 1, wherein the chemical herbicide is a triazine herbicide.

19. The weed control composition of claim 18, wherein the triazine herbicide is simetryne (chemical name: 2,4-bis(ethylamino)-6-methylthio-1,3,5-triazine).

20. A strain of *Drechslera monoceras*, which is free of pathogenicity against a crop under cultivation but has pathogenicity against *Echinochloa oryzicola*, *Echinochloa colonum, Echinochloa crus−galli var. formosensis*, *Echinochloa crus−galli var. crus−galli* and *Echinochloa crus−galli var. praticola* wherein the strain of Drechslera monoceras is MH-4418 (FERM BP-3414), MH-5011 (FERM BP-3415), MH-5017 (FERM BP-3411), MH-5018 (FERM BP-3412), MH-5511 (FERM BP-3417) or MH-9011 (FERM BP-3416).

21. A weed control agent comprising the strain of Drechslera monoceras of claim 20 and an agriculturally acceptable carrier.

**Patentansprüche**

1. Zusammensetzung zur Unkrautbekämpfung, umfassend einen Stamm von Drechslera spp., ein chemisches Herbizid und einen landwirtschaftlich verträglichen Träger, wobei der Stamm von Drechslera spp. keine Pathogenität gegenüber wirtschaftlich bedeutsamen Feldfrüchten, jedoch gegenüber Echinochloa spp. aufweist.

2. Zusammensetzung zur Unkrautbekämpfung nach Anspruch 1, wobei Drechslera spp. Drechslera monoceras ist.

3. Zusammensetzung zur Unkrautbekämpfung nach Anspruch 2, wobei der Stamm von Drechslera monoceras MH-0015 (FERM BP-2652), MH-2653 (FERM BP-2653), MH-2679 (FERM BP-2656), MH-1889 (FERM BP-3410), MH-4415 (FERM BP-3413), MH-4418 (FERM BP-3414), MH-5011 (FERM BP-3415), MH-5017 (FERM BP-3411), MH-5018 (FERM BP-3412), MH-5511 (FERM BP-3417) oder MH-9011 (FERM BP-3416) ist.

4. Zusammensetzung zur Unkrautbekämpfung nach Anspruch 1, wobei Drechslera spp. Drechslera ravenelii ist.

5. Zusammensetzung zur Unkrautbekämpfung nach Anspruch 4, wobei der Stamm von Drechslera ravenelii MH-0042 (FERM BP-2659), MH-0060 (FERM BP-2657) oder MH-2883 (FERM BP-3408) ist.

6. Zusammensetzung zur Unkrautbekämpfung nach Anspruch 1, wobei Drechslera spp. Drechslera poae ist.

7. Zusammensetzung zur Unkrautbekämpfung nach Anspruch 6, wobei der Stamm von Drechslera poae MH-0122 (FERM BP-2655), MH-2781 (FERM BP-3407) oder MH-2895 (FERM BP-3409) ist.

8. Zusammensetzung zur Unkrautbekämpfung nach Anspruch 1, wobei das chemische Herbizid ein Diphenylether-Herbizid ist.

9. Zusammensetzung zur Unkrautbekämpfung nach Anspruch 8, wobei das Diphenylether-Herbizid CNP (chemischer Name: 2,4,6-Trichlorphenyl-4-nitrophenylether), Chlormethoxynil (chemischer Name: 2,4-Dichlorphenyl-4-nitro-3-methoxyphenylether) oder Bifenox (chemischer Name: Methyl-5-(2,4-dichlorphenyl)-2-nitrobenzoat) ist.

10. Zusammensetzung zur Unkrautbekämpfung nach Anspruch 1, wobei das chemische Herbizid ein Anilid-Herbizid ist.

11. Zusammensetzung zur Unkrautbekämpfung nach Anspruch 10, wobei das Anilid-Herbizid Mefenacet (chemischer Name: 2-Benzothiazol-3-yloxy-N-methylacetanilid), Pretilachlor (chemischer Name: 2-Chlor-2',6'-diethyl-N-(n-propoxyethyl)acetanilid),Naproanilid (chemischer Name: 2-(2-Naphthoxy)-propionanilid), NSK-850 (chemischer Name: 2-Chlor-N-(3-methoxy-2-thienyl)methyl-2,6-dimethylanilid), Butachlor (chemischer Name: 2-Chlor-2',6'-diethyl-N-(butoxymethyl)acetanilid), Propanil (chemischer Name: 3',4'-Dichlorpropionanilid) oder Clomeprop (chemischer Name: 2-(2,4-Dichlor-3-methylphenoxy)-propionanilid) ist.

12. Zusammensetzung zur Unkrautbekämpfung nach Anspruch 1, wobei das chemische Herbizid ein Thiolcarbamat-Herbizid ist.

13. Zusammensetzung zur Unkrautbekämpfung nach Anspruch 12, wobei das Thiolcarbamat-Herbizid Benthiocarb (chemischer Name: S-(4-Chlorbenzyl)-N,N-diethylthiolcarbamat), Dimepiperat (chemischer Name: S-($\alpha,\alpha$-Dimethylbenzyl)-piperidin-1-carbathioat), Molinat (chemischer Name: S-Ethyl-N,N-hexamethylenthiolcarbamat) oder Esprocarb (chemischer Name: S-Benzyl-N-ethyl-N-(1,2-dimethylpropyl)-thiolcarbamat) ist.

14. Zusammensetzung zur Unkrautbekämpfung nach Anspruch 1, wobei das chemische Herbizid ein Diazin-Herbizid ist.

15. Zusammensetzung zur Unkrautbekämpfung nach Anspruch 14, wobei das Diazin-Herbizid Oxadiazon (chemischer Name: 5-tert.-Butyl-3-(2,4-dichlor-5-isopropoxyphenyl)-1,3,4-oxazol-2(3H)-on) oder Pyrazolat (chemischer Name: 4-(2,4-Dichlorbenzoyl)-1,3-dimethyl-1H-pyrazo1-5-yl-p-to-luolsulfonat) ist.

16. Zusammensetzung zur Unkrautbekämpfung nach Anspruch 1, wobei das chemische Herbizid ein Sulfonylharnstoff-Herbizid ist.

17. Zusammensetzung zur Unkrautbekämpfung nach Anspruch 16, wobei das Sulfonylharnstoff-Herbizid Bensulfuronmethyl (chemischer Name: Methyl-2-[3-(4,6-dimethoxypyrimidin-2-yl)ureidosulfonylmethyl]-benzoat) oder Pyrazosulfuronethyl (chemischer Name: Ethyl-5-[3-(4,6-dimethoxypyrimidin-2-yl)-ureidosulfonyl]-1-methylpyrazol-4-carboxylat) ist.

18. Zusammensetzung zur Unkrautbekämpfung nach Anspruch 1, wobei das chemische Herbizid ein Triazin-Herbizid ist.

19. Zusammensetzung zur Unkrautbekämpfung nach Anspruch 18, wobei das Triazin-Herbizid Simetryn (chemischer Name: 2,4-Bis(ethylamino)-6-methylthio-1,3,5-triazin) ist.

**20.** Stamm von Drechslera monoceras, der keine Pathogenität gegenüber einer kultivierten Feldfrucht, aber gegenüber Echinochloa oryzicola, Echinochloa colonum, Echinochloa crus-galli var. formosensis, Echinochloa crus-galli var. crus-galli und Echinochloa crus-galli var. praticola aufweist, wobei der Stamm von Drechslera monoceras MH-4418 (FERM BP-3414), MH-5011 (FERM BP-3415), MH-5017 (FERM BP-3411), MH-5018 (FERM BP-3412), MH-5511 (FERM BP-3417) oder MH-9011 (FERM BP-3416) ist.

**21.** Unkrautbekämpfungsmittel, umfassend den Drechslera monoceras-Stamm gemäß Anspruch 20 und einen landwirtschaftlich verträglichen Träger.

**Revendications**

**1.** Une composition pour lutter contre les mauvaises herbes, comprenant une souche de *Drechslera spp.*, un herbicide chimique et un support acceptable en agriculture, ladite souche de *Drechslera spp.* étant dépourvue de pathogénicité contre les cultures économiques mais ayant une pathogénicité contre *Echinochloa spp.*

**2.** La composition pour combattre les mauvaises herbes de la revendication 1, dans laquelle *Drechslera spp.* est *Drechslera monoceras*.

**3.** La composition pour combattre les mauvaises herbes de la revendication 2, dans laquelle la souche de *Drechslera monoceras* est MH-0015 (FERM BP-2652), MH-2653 (FERM BP-2653), MH-2679 (FERM BP-2656), MH-1889 (FERM BP-3410), MH-4415 (FERM BP-3413), MH-4418 (FERM BP-3414), MH-5011 (FERM BP-3415), MH-5017 (FERM BP-3411), MH-5018 (FERM BP-3412), MH-5511 (FERM BP-3417) ou MH-9011 (FERM BP-3416).

**4.** La composition pour combattre les mauvaises herbes de la revendication 1, dans laquelle *Drechslera spp.* est *Drechslera ravenelii*.

**5.** La composition pour combattre les mauvaises herbes de la revendication 4, dans laquelle la souche de *Drechslera ravenelii* est MH-0042 (FERM BP-2659), MH-0060 (FERM BP-2657) ou MH-2883 (FERM BP-3408).

**6.** La composition pour combattre les mauvaises herbes de la revendication 1, dans laquelle *Drechslera spp.* est *Drechslera poae*.

**7.** La composition pour combattre les mauvaises herbes de la revendication 6, dans laquelle la souche de *Drechslera poae* est MH-0122 (FERM BP-2655), MH-2781 (FERM BP-3407) ou MH-2895 (FERM BP-3409).

**8.** La composition pour combattre les mauvaises herbes de la revendication 1, dans laquelle l'herbicide chimique est un herbicide de type éther diphénylique.

**9.** La composition pour combattre les mauvaises herbes de la revendication 8, dans laquelle l'herbicide de type éther diphénylique est le CNP (nom chimique : éther 2,4,6-trichlorophényl-4-nitrophénylique),le chlorméthoxynil (nom chimique : éther 2,4-dichlorophényl-4-nitro-3-méthoxyphénylique) ou le bifénox (nom chimique : 5-(2,4-dichlorophényl)-2-nitrobenzoate de méthyle).

**10.** La composition pour combattre les mauvaises herbes de la revendication 1, dans laquelle l'herbicide chimique est un herbicide de type anilide.

**11.** La composition pour combattre les mauvaises herbes de la revendication 10, dans laquelle l'herbicide de type anilide est le méfénacet (nom chimique : 2-benzothiazole-3-yloxy-N-méthylacétanilide),le prétilachlor (nom chimique : 2-chloro-2',6'-diéthyl-N-(n-propoxyéthyl)acétanilide), le naproanilide (nom chimique : anilide 2-(2-naphtoxy)propionique), le NSK-850 (nom chimique : 2-chloro-N-(3-méthoxy-2-thiényl)méthyl-2,6-diméthylanilide), le butachlor (nom chimique : 2-chloro-2',6'-diéthyl-N-(butoxyméthyl) acétanilide), le propanil (nom chimique : 3',4'-dichloropropionanilide) ou le clomeprop (nom chimique : 2-(2,4-dichloro-3-méthylphénoxy)propionanilide).

**12.** La composition pour combattre les mauvaises herbes de la revendication 1, dans laquelle l'herbicide chimique est un herbicide de type thiolcarbamate.

**13.** La composition pour combattre les mauvaises herbes de la revendication 12, dans laquelle l'herbicide de type thiolcarbamate est le benthiocarb (nom chimique : S-(4-chlorobenzyl)-N,N-diéthylthiolcarbamate, le dimépipérate (nom chimique : S-($\alpha$,$\alpha$-diméthylbenzyl)-pipéridine-1-carbathioat), le molinate (nom chimique : S-éthyl-N,N-hexaméthylènethiolcarbamate) ou l'esprocarb (nom chimique : S-benzyl-N-éthyl-N-(1,2-diméthylpropyl)thiolcarbamate).

**14.** La composition pour combattre les mauvaises herbes de la revendication 1, dans laquelle l'herbicide chimique est un herbicide de type diazine.

**15.** La composition pour combattre les mauvaises herbes de la revendication 14, dans laquelle l'herbicide de type diazine est l'oxadiazon (nom chimique : 5-tert-butyl-3-(2,4-dichloro-5-isopropoxyphényl)-1,3,4-oxazole-2(3H)-one) ou le pyrazolate (nom chimique : p-toluènesulfonate de 4-(2,4-dichlorobenzoyl)-1,3-diméthyl-1H-pyrazole-5-yle).

**16.** La composition pour combattre les mauvaises herbes de la revendication 1, dans laquelle l'herbicide chimique est un herbicide de type sulfonylurée.

**17.** La composition pour combattre les mauvaises herbes de la revendication 16, dans laquelle l'herbicide de type sulfonylurée est le bensulfuron méthyle (nom chimique : 2-[3-(4,6-diméthoxypyrimidine-2-yl)-uréidosulfonylméthyl]benzoate de méthyle) ou le pyrazosulfuron éthyle (nom chimique : 5-[3-(4,6-diméthoxypyrimidine-2-yl) uréidosulfonyl]-1-méthylpyrazole-4-carboxylate d'éthyle).

**18.** La composition pour combattre les mauvaises herbes de la revendication 1, dans laquelle l'herbicide chimique est un herbicide de type triazine.

**19.** La composition pour combattre les mauvaises herbes de la revendication 18, dans laquelle l'herbicide de type triazine est la simétryne (nom chimique : 2,4-bis(éthylamino)-6-méthylthio-1,3,5-triazine).

**20.** Une souche de *Drechslera monoceras*, qui est dépourvue de pathogénicité contre une culture en cours, mais qui a une pathogénicité contre *Echinochloa orizicola*, *Echinochloa colonum*, *Echinochloa crus−galli var. formosensis*, *Echinochloa crus−galli var. crus−galli* et *Echinochloa crus−galli var. praticola*, laquelle souche de *Drechslera monoceras* est MH-4418 (FERM BP-3414), MH-5011 (FERM BP-3415), MH-5017 (FERM BP-3411), MH-5018 (FERM BP-3412), MH-5511 (FERM BP-3417) ou MH-9011 (FERM BP-3416).

**21.** Un agent pour combattre les mauvaises herbes comprenant la souche de *Drechslera monoceras* de la revendication 20 et un support acceptable en agriculture.